# EUROPEAN PATENT SPECIFICATION

(11) **EP 1 784 419 B1**
(45) Date of publication and mention of the grant of the patent: **24.10.2012**
(21) Application number: 05786178.3
(22) Date of filing: 01.09.2005
(51) Int. Cl.: C07K 14/22, A61K 39/095

(54) **DOMAINS AND EPITOPES OF MENINGOCOCCAL PROTEIN NMB1870**
DOMÄNEN UND EPITOPE VON MENINGOKOKALLEM PROTEIN NMB1870
DOMAINES ET EPITOPES DE PROTEINES MENINGOCOCCIQUES NMB1870

(30) Priority: 01.09.2004 GB 0419408
(43) Date of publication of application: 16.05.2007
(73) Proprietor: Novartis Vaccines and Diagnostics S.r.l., 53100 Siena (SI) (IT)
(72) Inventor: MASIGNANI, Vega, 53100 Siena (IT); SCARSELLI, Maria, 53100 Siena (IT); RAPPUOLI, Rino, 53100 Siena (IT); PIZZA, Mariagrazia, 53100 Siena (IT); GIULIANI, Marzia, 53100 Siena (IT); DI MARCELLO, Federica, 53100 Siena (IT); VEGGI, Daniele, 53100 Siena (IT); CIUCCHI, Laura, 53100 Siena (IT)
(74) Representative: Marshall, Cameron John
(86) International application number: PCT/IB2005/002968
(87) International publication number: WO 2006/024954

(56) References cited:
- WO-A-2004/048404
- GIULIANI MARZIA MONICA ET AL: "The region comprising amino acids 100 to 255 of Neisseria meningitidis lipoprotein GNA 1870 elicits bactericidal antibodies" INFECTION AND IMMUNITY, vol. 73, no. 2, February 2005 (2005-02), pages 1151-1160, XP002384460 ISSN: 0019-9567
- MASIGNANI V ET AL: "Vaccination against Neisseria meningitidis using three variants of the lipoprotein GNA1870" JOURNAL OF EXPERIMENTAL MEDICINE, TOKYO, JP, vol. 197, no. 6, 17 March 2003 (2003-03-17), pages 789-799, XP002286107 ISSN: 0022-1007 cited in the application

## Description

### TECHNICAL FIELD

This invention is in the field of immunisation and, in particular, immunisation against diseases caused by pathogenic bacteria in the genus *Neisseria,* such as *N.meningitidis* (meningococcus).

### BACKGROUND ART

*Neisseria meningitidis* is a Gram-negative encapsulated bacterium which colonises the upper respiratory tract of approximately 10% of human population. Although polysaccharide and conjugate vaccines are available against serogroups A, C, W135 and Y, this approach cannot be applied to serogroup B because the capsular polysaccharide is a polymer of polysialic acid, which is a self antigen in humans. To develop a vaccine against serogroup B, surface-exposed proteins contained in outer membrane vesicles (OMVs) have been used. These vaccines elicit serum bactericidal antibody responses and protect against disease, but they fail to induce cross-strain protection [1]. Some workers are therefore focusing on specific meningococcal antigens for use in vaccines.

One such antigen is 'NMB1870'. This protein was originally disclosed as protein '741' from strain MC58 [SEQ IDs 2535 & 2536 in ref. 2; SEQ ID 1 herein], and has also been referred to as 'GNA1870' [ref. 3, following ref. 4] and as 'ORF2086' [5,6]. This protein is expressed across all meningococcal serogroups and has been found in multiple meningococcal strains. NMB1870 sequences group into three families, and it has been found that serum raised against a given family is bactericidal within the same family, but is not active against strains which express one of the other two families i.e. there is intra-family cross-protection, but not inter-family cross-protection.

To achieve cross-strain protection using NMB1870, therefore, more than one family is used. To avoid the need to express and purify separate proteins, it has been proposed to express different families as hybrid proteins [7], including two or three of the families in a single polypeptide chain. Several hybrids have been tested and give encouraging anti-meningococcal efficacy.

It is an object of the invention to provide further and improved approaches for overcoming the family specificity of protection afforded by NMB1870, and to use these approaches for providing immunity against meningococcal disease and/or infection, particularly for serogroup B.

### DISCLOSURE OF THE INVENTION

The inventors have found that NMB1870 exposes some of its epitopes in surface loops situated between alpha helices. Substitution of loop epitopes from one family into the loop position in another family allows chimeric NMB1870 to be produced with multi-family antigenicity.

Thus the invention provides chimeric NMB1870 proteins that comprise portions of NMB1870 from different families as defined in the claims. Whereas each NMB1870 family can elicit antibodies (*e.g*. in mice) that are effective only against strains in the same NMB1870 family, chimeric polypeptides of the invention can elicit antibodies that recognise NMB1870 proteins from more than one family.

Bactericidal antibody responses are conveniently measured in mice and are a standard indicator of vaccine efficacy [*e.g*. see end-note 14 of reference 4]. Chimeric proteins can preferably elicit an antibody response which is bactericidal against at least one *N.meningitidis* strain from each of at least two of the following three groups of strains:
(I) MC58, gb185 (=M01-240185), m4030, m2197, m2937, iss1001, NZ394/98, 67/00, 93/114, bz198, m1390, nge28, lnp17592, 00-241341, f6124, 205900, m198/172, bz133, gb149 (=M01-240149), nm008, nm092, 30/00, 39/99, 72/00, 95330, bz169, bz83, cu385, h44/76, m1590, m2934, m2969, m3370, m4215, m4318, n44/89, 14847.
(II) 961-5945, 2996, 96217, 312294, 11327, a22, gb013 (=M01-240013), e32, m1090, m4287, 860800, 599, 95N477, 90-18311, c11, m986, m2671, 1000, m1096, m3279, bz232, dk353, m3697, ngh38, L93/4286.
(III) M1239, 16889, gb355 (=M01-240355), m3369, m3813, ngp165.

For example, a chimeric polypeptide can elicit a bactericidal response effective against two or more of serogroup B *N.meningitidis* strains MC58, 961-5945 and M1239.

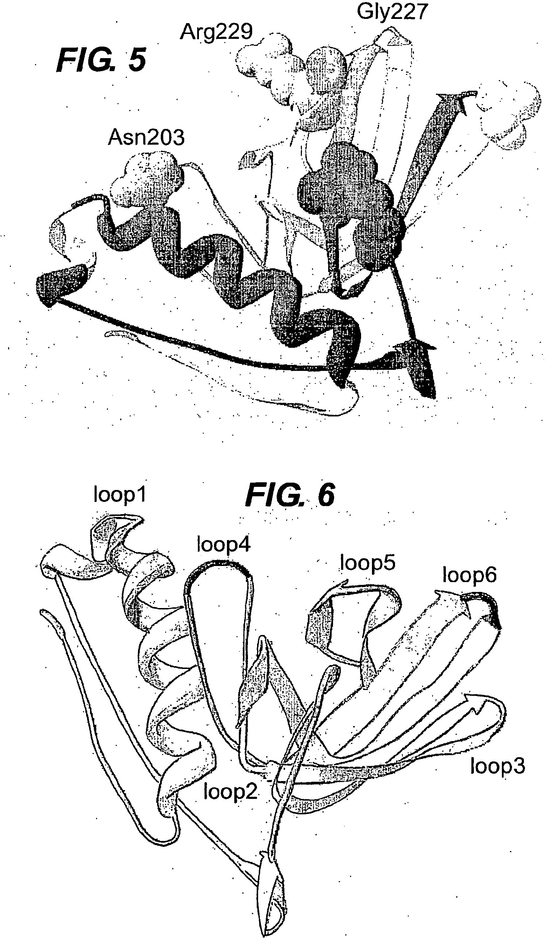

The chimeric polypeptide can preferably elicit an antibody response which is bactericidal against at least 50% of clinically-relevant meningococcal serogroup B strains (*e.g*. 60%, 70%, 80%, 90%, 95% or more). The chimeric polypeptide may elicit an antibody response which is bactericidal against strains of serogroup B *N.meningitidis* and strains of at least one (*e.g*. 1, 2, 3, 4) of serogroups A, C, W135 and Y. The chimeric polypeptide may elicit an antibody response which is bactericidal against strains of *N.gonococcus* and/or *N.cinerea.* The chimeric polypeptide may elicit an antibody response which is bactericidal against strains from at least two of the three main branches of the dendrogram shown in Figure 5 of reference 3.

The chimeric polypeptide may elicit an antibody response which is bactericidal against *N.meningitidis* strains in at least 2 (*e.g*. 2, 3, 4, 5, 6, 7) of hypervirulent lineages ET-37, ET-5, cluster A4, lineage 3, subgroup I, subgroup III, and subgroup IV-1 [8,9]. Chimeras may additionally induce bactericidal antibody responses against one or more hyperinvasive lineages.

Chimeras may elicit an antibody response which is bactericidal against *N.meningitidis* strains in at least at least 2 (*e.g*. 2, 3, 4, 5, 6, 7) of the following multilocus sequence types: ST1, ST4, ST5, ST8, ST11, ST32 and ST41 [10]. The chimera may also elicit an antibody response which is bactericidal against ST44 strains.

The composition need not induce bactericidal antibodies against each and every MenB strain within the specified lineages or MLST; rather, for any given group of four of more strains of serogroup B meningococcus within a particular hypervirulent lineage or MLST, the antibodies induced by the composition are bactericidal against at least 50% (*e.g.* 60%, 70%, 80%, 90% or more) of the group. Preferred groups of strains will include strains isolated in at least four of the following countries: GB, AU, CA, NO, IT, US, NZ, NL, BR, and CU. The serum preferably has a bactericidal titre of at least 1024 (*e.g.* 2¹⁰, 2¹¹, 2¹², 2¹³, 2¹⁴, 2¹⁵, 2¹⁶, 2¹⁷, 2¹⁸ or higher, preferably at least 2¹⁴) *i.e*. the serum is able to kill at least 50% of test bacteria of a particular strain when diluted 1:1024 *e.g*. as described in end-note 14 of reference 4. Preferred chimeric polypeptides can elicit an antibody response in mice that remains bactericidal even when the serum is diluted 1:4096 or further.

SEQ ID NO: 1 is the full-length family I NMB 1870 sequence from serogroup B strain MC58:

MNRTAFCCLSLTTALILTACSSGGGGVAADIGAGLADALTAPLDHKDKGLQSLTLDQSVRKNEKLK
LAAQGAEKTYGNGDSLNTGKLKNDKVSRFDFIRQIEVDGQLITLESGEFQVYKQSHSALTAFQTEQ
IQDSEHSGKMVAKRQFRIGDIAGEHTSFDKLPEGGRATYRGTAFGSDDAGGKLTYTIDFAAKQGNG
KIEHLKSPELNVDLAAADIKPDGKRHAVISGSVLYNQAEKGSYSLGIFGGKAQEVAGSAEVKTVNG
IRHIGLAAKQ

The N-terminus of the mature processed lipoprotein is underlined (Cys-20).

NMB1870 sequences fall into three families [3,7] that are referred to herein as families I, II and III. The prototypic sequences for families I-III are, respectively, SEQ ID NOS: 1-3. The phylogenetic and dendrogram methods of reference 3 can be followed in order to readily determine the family for any given NMB1870 sequence, and a pairwise alignment with each of the three prototypic NMB1870 sequences can also be used to find the closest family match. Sequences fall distinctly into the three families, with sequence identity being 74.1% between families I & II, 62.8% between families I & III and 84.7% between families II & III, and with sequence variation within each family being low (*e.g*. a minimum of 91.6% identity in family I, 93.4% in family II and 93.2% in family III). As a quick way of determining a sequence's family without requiring a phylogenetic analysis, a sequence can be placed in family I if it has at least 85% sequence identity to SEQ ID NO: 1, can be placed in family II if it has at least 85% sequence identity to SEQ ID NO: 2, and can be placed in family III if it has at least 85% sequence identity to SEQ ID NO: 3.

### NMB1870 surface loops

The surface loops of SEQ ID NO: 1, lying between alpha helices, are: (1) amino acids 134-141; (2) amino acids 162-168; (3) amino acids 181-182; (4) amino acid 197; (5) amino acids 219-223; (6) amino acids 234-236; (7) amino acids 261-267:

MNRTAFCCLSLTTALILTACSSGGGGVAADIGAGLADALTAPLDHKDKGLQSLTLDQSVRKNEKLK
LAAQGAEKTYGNGDSLNTGKLKNDKVSRFDFIRQIEVDGQLITLESGEFQVYKQSHSALTAFQTEQ
IQDSEHSGKMVAKRQFRIGDIAGEHTSFDKLPEGGRATYRGTAFGSDDAGGKLTYTIDFAAKQGNG
KIEHLKSPELNVDLAAADIKPDGKRHAVISGSVLYNQAEKGSYSLGIFGGKAQEVAGSAEVKTVNG
IRHIGLAAKQ

By aligning SEQ ID NO: 1 with any other NMB1870 sequence, the skilled person can identify the positions of loops (1) to (7) in that sequence. For ease of reference, however, the coordinates of a loop are defined herein as the string of amino acid(s) in a NMB 1870 sequence that, when aligned to SEQ ID NO: 1 using a pairwise alignment algorithm, starts with the amino acid aligned to the first amino acid residue of the loop defined above in SEQ ID NO: 1 above and ends with the last amino acid of the loop defined above in SEQ ID NO: 1.

### Constructing chimeric NMB1870 sequences

The invention provides a process for producing a chimeric NMB1870 amino acid sequence, as defined in claim 1.

Steps (b) to (d) may be performed more than once for the same alignment from step (a) *i.e*. multiple substitutions from the second sequence into the first sequence can be performed. Similarly, steps (a) to (d) may be performed more than once, with a different "second amino acid sequence" optionally being used during subsequent steps (a) *i.e.* a first sequence can be aligned with a second sequence and subjected to the substitution procedure, and then may be aligned with a different second sequence and subjected to a further substitution, *etc*.

Thus the invention provides a process for producing a chimeric NMB1870 amino acid sequence, comprising the steps of: (a) aligning a first NMB1870 amino acid sequence with a second NMB1870 amino acid sequence, to give a first pair of aligned sequences; (b) selecting a portion of the first amino acid sequence, starting at amino acid *a₁* of said first amino acid sequence and ending at amino acid *b₁* of said first amino acid sequence; (c) selecting a portion of the second amino acid sequence, starting at amino acid *a₂* of said second amino acid sequence and ending at amino acid *b₂* of said second amino acid sequence, wherein residues *a₁* & *a₂* and *b₁* & *b₂* are aligned in the first pair of aligned sequences; (d) replacing said portion of the first amino acid sequence with said portion of the second amino acid sequence, thereby providing an intermediate chimeric NMB1870 amino acid sequence; (e) aligning the first NMB1870 amino acid sequence, or the intermediate chimeric sequence, with a third NMB1870 amino acid sequence, to give a second pair of aligned sequences; (f) selecting a portion of the intermediate chimeric sequence, starting at amino acid *a₃* of the intermediate chimeric sequence, and ending at amino acid *b₃ of* the intermediate chimeric sequence; (g) selecting a portion of the third amino acid sequence, starting at amino acid *a₄* of said third amino acid sequence and ending at amino acid *b₄* of said third amino acid sequence, wherein residues *a₃* & *a₄* and *b₃* & *b₄* are aligned in the second pair of aligned sequences; and (h) replacing said portion of the intermediate chimeric sequence, with said portion of the third amino acid sequence, thereby providing the chimeric NMB1870 amino acid sequence.

The selected portions are preferably at least 3 amino acids long.

The substituted sequence(s) are surface loop sequences. The invention includes situations including up to 7 substitutions.

The invention also provides a polypeptide comprising a chimeric NMB1870 amino acid sequence, wherein said chimeric NMB 1870 amino acid sequence is obtainable by the above process.

The process of the invention may be followed by the further step of producing a polypeptide comprising said chimeric NMB1870 amino acid sequence *e.g*. by recombinant protein expression.

### Loop substitution

The inventors have found that NMB1870 exposes some of its epitopes in surface loops situated between alpha helices. Substitution of loop epitopes from one family into the loop position in another family allows chimeric NMB1870 to be produced with multi-family antigenicity.

Thus the invention provides a polypeptide comprising a modified amino acid sequence of a first family of NMB1870, wherein the modified sequence includes at least one (*e.g*. 1, 2, 3, 4, 5, 6 or 7) surface loop sequence from a second family of NMB1870 in place of a surface loop sequence from the first family.

The invention also provides a polypeptide comprising an amino acid sequence:

-B₁-L₁-B₂-L₂-B₃-L₃-B₄-L₄-B₅-L₅-B₆-L₆-B₇-L₇-B₈-

wherein: (a) each of said B₁, B₂, B₃, B₄, B₅, B₆, B₇ and B₈ is: (i) a fragment of SEQ ID NO:1; (ii) an amino acid sequence having at least *m*% sequence identity to said fragment of (i) and/or comprising a fragment of at least *mm* contiguous amino acids from said fragment of (i); (b) each of said L₁, L₂, L₃, L₄, L₅, L₆ and L₇ is: (iii) a fragment of SEQ ID NO: 1, SEQ ID NO: 2 and/or of SEQ ID NO: 3; , provided that at least one of said L₁, L₂, L₃, L₄, L₅, L₆ and L₇ is not a fragment of SEQ ID NO:1

Thus the polypeptide comprises a basic backbone sequence, in eight parts, and seven loops, one between each consecutive part of backbone sequence, but at least one of the loop sequences is taken from a NMB 1870 sequence that is from a different NMB1870 family from the basic backbone sequence. It is preferred to use surface loops from more than one different NMB1870 sequences, and it is possible to insert these loops into a single backbone sequence.

The meaning of "(i) a fragment of SEQ ID NO:1" is as follows:

| | **Amino acid co-ordinates within SEQ ID NO:1** | | | | | | | |
|---|---|---|---|---|---|---|---|---|
| | B₁ | B₂ | B₃ | B₄ | B₅ | B₆ | B₇ | B₈ |
| **1** | 1-133 | 142-161 | 169-180 | 183-196 | 198-218 | 224-233 | 237-260 | 268-274 |

Similarly, "(iii) a fragment of SEQ ID NO: 1, SEQ ID NO: 2 and/or of SEQ ID NO: 3" is defined as follows:

| | **Amino acid co-ordinates within SEQ ID NO: 1, 2 or 3** | | | | | | |
|---|---|---|---|---|---|---|---|
| ***SEQ*** | L₁ | L₂ | L₃ | L₄ | L₅ | L₆ | L₇ |
| **1** | 134-141 | 162-168 | 181-182 | 197 | 219-223 | 234-236 | 261-267 |
| **2** | 134-141 | 162-167 | 180-181 | 196 | 218-222 | 233-235 | 260-266 |
| **3** | 142-149 | 170-175 | 188-189 | 204 | 226-230 | 241-243 | 268-274 |

For example, the invention provides a polypeptide comprising an amino acid sequence:

-B₁-L₁-B₂-L₂-B₃-L₃-B₄-L₄-B₅-L₅-B₆-L₆-B₇-L₇-B₈-

wherein: B₁ is amino acids 1-133 of SEQ ID NO: 1, or an amino acid sequence having at least 95% sequence identity to said amino acids 1-133 and/or comprising a fragment of at least 6 contiguous amino acids from said amino acids 1-133; B₂ is amino acids 142-161 of SEQ ID NO: 1, or an amino acid sequence having at least 95% sequence identity to said amino acids 142-161 and/or comprising a fragment of at least 6 contiguous amino acids from said amino acids 142-161 ... B₇ is amino acids 268-274 of SEQ ID NO: 1 L₁ is amino acids 134-141 of SEQ ID NO: 2; L₂ is amino acids 162-167 of SEQ ID NO: 2 ... L₇ is amino acids 268-274 of SEQ ID NO: 3 *etc*.

The invention provides a polypeptide comprising an amino acid sequence that has an overall sequence identity to SEQ ID NO: *Q* of 80%, wherein: the value of *q* is at least *r*; the sequence identity of said amino acid sequence to SEQ ID NO: *Q* is more than 80% at the backbone regions of SEQ ID NO: *Q*; and the sequence identity of said amino acid sequence to SEQ ID NO: *Q* is less than 80% at the loop regions of SEQ ID NO: *Q*.

The value of *Q* is 1, 2 or 3, and the boundaries of the loop regions and of the backbone regions are selected accordingly from the above tables (L₁ to L₇ being the loops, and B₁ to B₈ being the backbone).

### Polypeptides

The invention provides the polypeptides described above.

NMB1870 is naturally a lipoprotein in *N.meningitidis.* It has also been found to be lipidated when expressed in *E.coli.* Preferred polypeptides of the invention have a C-terminus cysteine residue, which may be lipidated *e.g*. comprising a palmitoyl group.

A characteristic of preferred polypeptides of the invention is the ability to induce bactericidal anti-meningococcal antibodies after administration to a host animal.

Polypeptides of the invention can be prepared by various means *e.g*. by chemical synthesis (at least in part), by digesting longer polypeptides using proteases, by translation from RNA, by purification from cell culture *(e.g.* from recombinant expression or from *N.meningitidis* culture). *etc.* Heterologous expression in an *E.coli* host is a preferred expression route (*e.g*. in DH5a, BL21(DE₃), BLR, *etc*.).

Polypeptides of the invention may be attached or immobilised to a solid support.

Polypeptides of the invention may comprise a detectable label *e.g*. a radioactive label, a fluorescent label, or a biotin label. This is particularly useful in immunoassay techniques.

Polypeptides can take various forms (*e.g*. native, fusions, glycosylated, non-glycosylated, lipidated, disulfide bridges, *etc.).*

Polypeptides are, preferably prepared in substantially pure or substantially isolated form (*i.e.* substantially free from other Neisserial or host cell polypeptides) or substantially isolated form. In general, the polypeptides are provided in a non-naturally occurring environment *e.g.* they are separated from their naturally-occurring environment. In certain embodiments, the subject polypeptide is present in a composition that is enriched for the polypeptide as compared to a control. As such, purified polypeptide is provided, whereby purified is meant that the polypeptide is present in a composition that is substantially free of other expressed polypeptides, where by substantially free is meant that less than 90%, usually less than 60% and more usually less than 50% of the composition is made up of other expressed polypeptides.

The term "polypeptide" refers to amino acid polymers of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc.),* as well as other modifications known in the art. Polypeptides can occur as single chains or associated chains.

### Nucleic acids

The invention provides nucleic acid encoding a polypeptide of the invention as defined above. The invention also provides nucleic acid comprising: (a) a fragment of at least **n** consecutive nucleotides from said nucleic acid, wherein *n* is 10 or more (*e.g*. 12, 14, 15, 18, 20, 25, 30, 35, 40, 50, 60, 70, 80, 90, 100, 150, 200, 500 or more); and/or (b) a sequence having at least 50% *(e.g.* 60%, 70%, 80%, 90%, 95%, 96%, 97%, 98%, 99% or more) sequence identity to said nucleic acid.

Furthermore, the invention provides nucleic acid which can chimericise to nucleic acid encoding a polypeptide of the invention, preferably under "high stringency" conditions (*e.g*. 65°C in a 0.1xSSC, 0.5% SDS solution).

Nucleic acids of the invention can be used in hybridisation reactions (*e.g.* Northern or Southern blots, or in nucleic acid microarrays or 'gene chips') and amplification reactions (*e.g*. PCR, SDA, SSSR, LCR, TMA, NASBA, *etc.)* and other nucleic acid techniques.

Nucleic acids of the invention may be prepared in many ways *e.g.* by chemical synthesis (*e.g.* phosphoramidite synthesis of DNA) in whole or in part, by digesting longer nucleic acids using nucleases (*e.g*. restriction enzymes), by joining shorter nucleic acids or nucleotides (*e.g*. using ligases or polymerases), from genomic or cDNA libraries, *etc.*

Nucleic acids of the invention can take various forms *e.g*. single-stranded, double-stranded, vectors, primers, probes, labelled, unlabelled, *etc.*

Nucleic acids of the invention are preferably in isolated or substantially isolated form.

The invention includes nucleic acid comprising sequences complementary to those described above *e.g*. for antisense or probing, or for use as primers.

The term "nucleic acid" includes DNA and RNA, and also their analogues, such as those containing modified backbones, and also peptide nucleic acids (PNA), *etc.*

Nucleic acid according to the invention may be labelled *e.g*. with a radioactive or fluorescent label. This is particularly useful where the nucleic acid is to be used in nucleic acid detection techniques *e*.*g*. where the nucleic acid is a primer or as a probe for use in techniques such as PCR, LCR, TMA, NASBA, *etc.*

The invention also provides vectors comprising nucleotide sequences of the invention (*e.g*. cloning or expression vectors, such as those suitable for nucleic acid immunisation) and host cells transformed with such vectors.

### Immunisation

Polypeptides of the invention are preferably provided as immunogenic compositions, and the invention provides an immunogenic composition of the invention for use as a medicament.

Compositions of the invention can also be used in a method for raising an antibody response in a mammal, comprising administering an immunogenic composition of the invention to the mammal. The antibody response is preferably a protective and/or bactericidal antibody response.

Compositions of the invention can also be used in a method for protecting a mammal against a Neisserial (*e.g*. meningococcal) infection, comprising administering to the mammal an immunogenic composition of the invention.

The invention provides chimeric polypeptides of the invention for use as medicaments (*e.g*. as immunogenic compositions or as vaccines)

The mammal is preferably a human. The human may be an adult or, preferably, a child. Where the vaccine is for prophylactic use, the human is preferably a child (*e.g*. a toddler or infant); where the vaccine is for therapeutic use, the human is preferably an adult. A vaccine intended for children may also be administered to adults *e.g.* to assess safety, dosage, immunogenicity, *etc*.

The uses and methods are particularly useful for preventing/treating diseases including, but not limited to, meningitis (particularly bacterial meningitis) and bacteremia.

Efficacy of therapeutic treatment can be tested by monitoring Neisserial infection after administration of the composition of the invention. Efficacy of prophylactic treatment can be tested by monitoring immune responses against NMB1870 after administration of the composition. Immunogenicity of compositions of the invention can be determined by administering them to test subjects (*e.g*. children 12-16 months age, or animal models [13]) and then determining standard parameters including serum bactericidal antibodies (SBA) and ELISA titres (GMT). These immune responses will generally be determined around 4 weeks after administration of the composition, and compared to values determined before administration of the composition. A SBA increase of at least 4-fold or 8-fold is preferred. Where more than one dose of the composition is administered, more than one post-administration determination may be made.

Preferred compositions of the invention can confer an antibody titre in a patient that is superior to the criterion for seroprotection for each antigenic component for an acceptable percentage of human subjects. Antigens with an associated antibody titre above which a host is considered to be seroconverted against the antigen are well known, and such titres are published by organisations such as WHO. Preferably more than 80% of a statistically significant sample of subjects is seroconverted, more preferably more than 90%, still more preferably more than 93% and most preferably 96-100%.

Compositions of the invention will generally be administered directly to a patient. Direct delivery may be accomplished by parenteral injection (*e.g*. subcutaneously, intraperitoneally, intravenously, intramuscularly, or to the interstitial space of a tissue), or by rectal, oral, vaginal, topical, transdermal, intranasal, ocular, aural, pulmonary or other mucosal administration. Intramuscular administration to the thigh or the upper arm is preferred. Injection may be via a needle (*e.g*. a hypodermic needle), but needle-free injection may alternatively be used. A typical intramuscular dose is 0.5 ml.

The invention may be used to elicit systemic and/or mucosal immunity.

Dosage treatment can be a single dose schedule or a multiple dose schedule. Multiple doses may be used in a primary immunisation schedule and/or in a booster immunisation schedule. A primary dose schedule may be followed by a booster dose schedule. Suitable timing between priming doses (*e.g*. between 4-16 weeks), and between priming and boosting, can be routinely determined.

The immunogenic composition of the invention will generally include a pharmaceutically acceptable carrier, which can be any substance that does not itself induce the production of antibodies harmful to the patient receiving the composition, and which can be administered without undue toxicity. Suitable carriers can be large, slowly-metabolised macromolecules such as proteins, polysaccharides, polylactic acids, polyglycolic acids, polymeric amino acids, amino acid copolymers, and inactive virus particles. Such carriers are well known to those of ordinary skill in the art. Pharmaceutically acceptable carriers can include liquids such as water, saline, glycerol and ethanol. Auxiliary substances, such as wetting or emulsifying agents, pH buffering substances, and the like, can also be present in such vehicles. Liposomes are suitable carriers. A thorough discussion of pharmaceutical carriers is available in ref. 14.

Neisserial infections affect various areas of the body and so the compositions of the invention may be prepared in various forms. For example, the compositions may be prepared as injectables, either as liquid solutions or suspensions. Solid forms suitable for solution in, or suspension in, liquid vehicles prior to injection can also be prepared. The composition may be prepared for topical administration *e.g.* as an ointment, cream or powder. The composition be prepared for oral administration *e.g.* as a tablet or capsule, or as a syrup (optionally flavoured). The composition may be prepared for pulmonary administration *e.g.* as an inhaler, using a fine powder or a spray. The composition may be prepared as a suppository or pessary. The composition may be prepared for nasal, aural or ocular administration *e.g.* as drops.

The composition is preferably sterile. It is preferably pyrogen-free. It is preferably buffered e.g. at between pH 6 and pH 8, generally around pH 7. Where a composition comprises an aluminium hydroxide salt, it is preferred to use a histidine buffer [15]. Compositions of the invention may be isotonic with respect to humans.

Immunogenic compositions comprise an immunologically effective amount of immunogen, as well as any other of other specified components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g*. non-human primate, primate, *etc*.), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. Dosage treatment may be a single dose schedule or a multiple dose schedule (*e.g*. including booster doses). The composition may be administered in conjunction with other immunoregulatory agents.

Adjuvants which may be used in compositions of the invention include, but are not limited to:

### A. Mineral-containing compositions

Mineral containing compositions suitable for use as adjuvants in the invention include mineral salts, such as aluminium salts and calcium salts. The invention includes mineral salts such as hydroxides (*e.g*. oxyhydroxides), phosphates (*e.g*. hydroxyphosphates, orthophosphates), sulphates, *etc.* [*e.g*. see chapters 8 & 9 of ref. 16], or mixtures of different mineral compounds, with the compounds taking any suitable form (*e.g.* gel, crystalline, amorphous, *etc.),* and with adsorption being preferred. The mineral containing compositions may also be formulated as a particle of metal salt [17].

Aluminium phosphates are particularly preferred, particularly in compositions which include a *H.influenzae* saccharide antigen, and a typical adjuvant is amorphous aluminium hydroxyphosphate with PO₄/Al molar ratio between 0.84 and 0.92, included at 0.6mg Al³⁺/ml. Adsorption with a low dose of aluminium phosphate may be used *e.g*. between 50 and 100µg Al³⁺ per conjugate per dose. Where there is more than one conjugate in a composition, not all conjugates need to be adsorbed.

### B. Oil Emulsions

Oil emulsion compositions suitable for use as adjuvants in the invention include squalene-water emulsions, such as MF59 [Chapter 10 of ref. 16; see also ref. 18] (5% Squalene, 0.5% Tween 80, and 0.5% Span 85, formulated into submicron particles using a microfluidizer). Complete Freund's adjuvant (CFA) and incomplete Freund's adjuvant (IFA) may also be used.

### C. Saponin formulations [chapter 22 of ref. 16]

Saponin formulations may also be used as adjuvants in the invention. Saponins are a heterologous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs. QS21 is marketed as Stimulon™.

Saponin compositions have been purified using HPLC and RP-HPLC. Specific purified fractions using these techniques have been identified, including QS7, QS17, QS18, QS21, QH-A, QH-B and QH-C. Preferably, the saponin is QS21. A method of production of QS21 is disclosed in ref. 19. Saponin formulations may also comprise a sterol, such as cholesterol [20].

Combinations of saponins and cholesterols can be used to form unique particles called immunostimulating complexs (ISCOMs) [chapter 23 of ref. 16]. ISCOMs typically also include a phospholipid such as phosphatidylethanolamine or phosphatidylcholine. Any known saponin can be used in ISCOMs. Preferably, the ISCOM includes one or more of QuilA, QHA & QHC. ISCOMs are further described in refs. 20-22. Optionally, the ISCOMS may be devoid of additional detergent [23].

A review of the development of saponin based adjuvants can be found in refs. 24 & 25.

### D. Virosomes and virus-like particles

Virosomes and virus-like particles (VLPs) can also be used as adjuvants in the invention. These structures generally contain one or more proteins from a virus optionally combined or formulated with a phospholipid. They are generally non-pathogenic, non-replicating and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qß-phage (such as coat proteins), GA-phage, fr-phage, AP205 phage, and Ty (such as retrotransposon Ty protein p1). VLPs are discussed further in refs. 26-31. Virosomes are discussed further in, for example, ref. 32

### E. Bacterial or microbial derivatives

Adjuvants suitable for use in the invention include bacterial or microbial derivatives such as non-toxic derivatives of enterobacterial lipopolysaccharide (LPS), Lipid A derivatives, immunostimulatory oligonucleotides and ADP-ribosylating toxins and detoxified derivatives thereof.

Non-toxic derivatives of LPS include monophosphoryl lipid A (MPL) and 3-O-deacylated MPL (3dMPL). 3dMPL is a mixture of 3 de-O-acylated monophosphoryl lipid A with 4, 5 or 6 acylated chains. A preferred "small particle" form of 3 De-O-acylated monophosphoryl lipid A is disclosed in ref. 33. Such "small particles" of 3dMPL are small enough to be sterile filtered through a 0.22µm membrane [33]. Other non-toxic LPS derivatives include monophosphoryl lipid A mimics, such as aminoalkyl glucosaminide phosphate derivatives *e.g*. RC-529 [34,35].

Lipid A derivatives include derivatives of lipid A from *Escherichia coli* such as OM-174. OM-174 is described for example in refs. 36 & 37.

Immunostimulatory oligonucleotides suitable for use as adjuvants in the invention include nucleotide sequences containing a CpG motif (a dinucleotide sequence containing an unmethylated cytosine linked by a phosphate bond to a guanosine). Double-stranded RNAs and oligonucleotides containing palindromic or poly(dG) sequences have also been shown to be immunostimulatory.

The CpG's can include nucleotide modifications/analogs such as phosphorothioate modifications and can be double-stranded or single-stranded. References 38, 39 and 40 disclose possible analog substitutions *e.g*. replacement of guanosine with 2'-deoxy-7-deazaguanosine. The adjuvant effect of CpG oligonucleotides is further discussed in refs. 41-46.

The CpG sequence may be directed to TLR9, such as the motif GTCGTT or TTCGTT [47]. The CpG sequence may be specific for inducing a Th1 immune response, such as a CpG-A ODN, or it may be more specific for inducing a B cell response, such a CpG-B ODN. CpG-A and CpG-B ODNs are discussed in refs. 48-50. Preferably, the CpG is a CpG-A ODN.

Preferably, the CpG oligonucleotide is constructed so that the 5' end is accessible for receptor recognition. Optionally, two CpG oligonucleotide sequences may be attached at their 3' ends to form "immunomers". See, for example, refs. 47 & 51-53.

Bacterial ADP-ribosylating toxins and detoxified derivatives thereof may be used as adjuvants in the invention. Preferably, the protein is derived from *E.coli* (*E.coli* heat labile enterotoxin "LT"), cholera ("CT"), or pertussis ("PT"). The use of detoxified ADP-ribosylating toxins as mucosal adjuvants is described in ref. 54 and as parenteral adjuvants in ref. 55. The toxin or toxoid is preferably in the form of a holotoxin, comprising both A and B subunits. Preferably, the A subunit contains a detoxifying mutation; preferably the B subunit is not mutated. Preferably, the adjuvant is a detoxified LT mutant such as LT-K63, LT-R72, and LT-G192. The use of ADP-ribosylating toxins and detoxified derivaties thereof, particularly LT-K63 and LT-R72, as adjuvants can be found in refs. 56-63. Numerical reference for amino acid substitutions is preferably based on the alignments of the A and B subunits of ADP-ribosylating toxins set forth in ref. 64, specifically incorporated herein by reference in its entirety.

### F. Human immunomodulators

Human immunomodulators suitable for use as adjuvants in the invention include cytokines, such as interleukins (*e.g*. IL-1, IL-2, IL-4, IL-5, IL-6, IL-7, IL-12 [65], *etc.)* [66], interferons (*e.g*. interferon-γ), macrophage colony stimulating factor, and tumor necrosis factor.

### G. Bioadhesives and Mucoadhesives

Bioadhesives and mucoadhesives may also be used as adjuvants in the invention. Suitable bioadhesives include esterified hyaluronic acid microspheres [67] or mucoadhesives such as cross-linked derivatives of poly(acrylic acid), polyvinyl alcohol, polyvinyl pyrollidone, polysaccharides and carboxymethylcellulose. Chitosan and derivatives thereof may also be used as adjuvants in the invention [68].

### H. Microparticles

Microparticles may also be used as adjuvants in the invention. Microparticles (*i.e*. a particle of ~100nm to ~150µm in diameter, more preferably ~200nm to ~30µm in diameter, and most preferably ~500nm to ~10µm in diameter) formed from materials that are biodegradable and non-toxic (*e.g*. a poly(a-hydroxy acid), a polyhydroxybutyric acid, a polyorthoester, a polyanhydride, a polycaprolactone, *etc*.), with poly(lactide-co-glycolide) are preferred, optionally treated to have a negatively-charged surface (*e.g*. with SDS) or a positively-charged surface (*e.g*. with a cationic detergent, such as CTAB).

### I. Liposomes (Chapters 13 & 14 of ref. 16)

Examples of liposome formulations suitable for use as adjuvants are described in refs. 69-71.

### J. Polyoxyethylene ether and polyoxyethylene ester formulations

Adjuvants suitable for use in the invention include polyoxyethylene ethers and polyoxyethylene esters [72]. Such formulations further include polyoxyethylene sorbitan ester surfactants in combination with an octoxynol [73] as well as polyoxyethylene alkyl ethers or ester surfactants in combination with at least one additional non-ionic surfactant such as an octoxynol [74]. Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

### K. Polyphosphazene (PCPP)

PCPP formulations are described, for example, in refs. 75 and 76.

### L. Muramyl peptides

Examples of muramyl peptides suitable for use as adjuvants in the invention include N-acetyl-muramyl-L-threonyl-D-isoglutamine (thr-MDP), N-acetyl-normuramyl-L-alanyl-D-isoglutamine (nor-MDP), and N-acetylmuramyl-L-alanyl-D-isoglutaminyl-L-alanine-2-(1'-2'-dipalmitoyl-sn-glycero-3-hydroxyphosphoryloxy)-ethylamine MTP-PE).

### M. Imidazoquinolone Compounds.

Examples of imidazoquinolone compounds suitable for use adjuvants in the invention include Imiquamod and its homologues (*e,g*. "Resiquimod 3M"), described further in refs. 77 and 78.

The invention may also comprise combinations of aspects of one or more of the adjuvants identified above. For example, the following adjuvant compositions may be used in the invention: (1) a saponin and an oil-in-water emulsion [79]; (2) a saponin (*e.g.* QS21) + a non-toxic LPS derivative (*e.g.* 3dMPL) [80]; (3) a saponin (*e.g*. QS21) + a non-toxic LPS derivative (*e.g*. 3dMPL) + a cholesterol; (4) a saponin (*e.g.* QS21) + 3dMPL + IL-12 (optionally + a sterol) [81]; (5) combinations of 3dMPL with, for example, QS21 and/or oil-in-water emulsions [82]; (6) SAF, containing 10% squalane, 0.4% Tween 80™, 5% pluronic-block polymer L121, and thr-MDP, either microfluidized into a submicron emulsion or vortexed to generate a larger particle size emulsion. (7) Ribi^{™} adjuvant system (RAS), (Ribi Immunochem) containing 2% squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); and (8) one or more mineral salts (such as an aluminum salt) + a non-toxic derivative of LPS (such as 3dMPL).

Other substances that act as immunostimulating agents are disclosed in chapter 7 of ref. 16.

Aluminium salts (aluminium phosphates and particularly hydroxyphosphates, and/or hydroxides and particularly oxyhydroxide) and MF59 are preferred adjuvants for parenteral immunisation. Toxin mutants are preferred mucosal adjuvants. QS21 is another useful adjuvant for NMB1870, which may be used alone or in combination with one or more other adjuvants e.g. with an aluminium salt. Muramyl peptides include N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-hydroxyphosphoryloxy)-ethylamine MTP-PE), *etc.*

### Further antigenic components

Compositions of the invention include chimeric NMB1870 polypeptides. It is particularly preferred that the composition should not include complex or undefined mixtures of antigens *e.g*. it is preferred not to include outer membrane vesicles in the composition. Polypeptides of the invention are preferably expressed recombinantly in a heterologous host and then purified.

The composition of the invention includes a chimeric NMB1870 polypeptide. It may also include one or more further neisserial antigen(s), as a vaccine which targets more than one antigen per bacterium decreases the possibility of selecting escape mutants. Neisserial antigens for inclusion in the compositions include proteins comprising:
(a) the 446 even SEQ IDs (*i.e.* 2, 4, 6, ... , 890, 892) disclosed in reference 83.
(b) the 45 even SEQ IDs (i.e. 2, 4, 6, ... , 88, 90) disclosed in reference 84;
(c) the 1674 even SEQ IDs 2-3020, even SEQ IDs 3040-3114, and all SEQ IDs 3115-3241, disclosed in reference 2;
(d) the 2160 amino acid sequences NMB0001 to NMB2160 from reference 4;
(e) a meningococcal PorA protein, of any subtype, preferably recombinantly expressed;
(f) a variant, homolog, ortholog, paralog, mutant *etc.* of (a) to (e); or
(g) an outer membrane vesicle preparation from *N.meningitidis* [*e.g.* see ref. 177].

In addition to Neisserial protein antigens, the composition may include antigens for immunising against other diseases or infections. For example, the composition may include one or more of the following further antigens:
- a saccharide antigen from *N.meningitidis* serogroup A, C, W135 and/or Y, such as the oligosaccharide disclosed in ref. 85 from serogroup C [see also ref. 86] or the oligosaccharides of ref. 87.
- a saccharide antigen from *Streptococcus pneumoniae* [*e.g.* 8 8, 89, 90].
- an antigen from hepatitis A virus, such as inactivated virus [*e.g*. 91, 92].
- an antigen from hepatitis B virus, such as the surface and/or core antigens [*e.g.* 92, 93].
- a diphtheria antigen, such as a diphtheria toxoid [*e.g.* chapter 3 of ref. 94] *e.g.* the CRM₁₉₇ mutant [*e.g*. *95*].
- a tetanus antigen, such as a tetanus toxoid [*e.g*. chapter 4 of ref. 94].
- an antigen from *Bordetella pertussis,* such as pertussis holotoxin (PT) and filamentous haemagglutinin (FHA) from *B.pertussis*, optionally also in combination with pertactin and/or agglutinogens 2 and 3 [*e.g*. refs. 96 & 97].
- a saccharide antigen from *Haemophilus influenzae* B [*e.g*. 86].
- polio antigen(s) [*e.g*. 98, 99] such as IPV.
- measles, mumps and/or rubella antigens [e.g. chapters 9, 10 & 11 of ref. 94].
- influenza antigen(s) [*e.g.* chapter 19 of ref. 94], such as the haemagglutinin and/or neuraminidase surface proteins.
- an antigen from *Moraxella catarrhalis* [*e.g.* 100].
- an protein antigen from *Streptococcus agalactiae* (group B streptococcus) [*e.g*. 101, 102].
- a saccharide antigen from *Streptococcus agalactiae* (group B streptococcus).
- an antigen from *Streptococcus pyogenes* (group A streptococcus) [*e.g.* 102, 103, 104].
- an antigen from *Staphylococcus aureus* [*e.g.* 105].

The composition may comprise one or more of these further antigens.

Toxic protein antigens may be detoxified where necessary (*e.g*. detoxification of pertussis toxin by chemical and/or genetic means [97]).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens. DTP combinations are thus preferred.

Saccharide antigens are preferably in the form of conjugates. Carrier proteins for the conjugates include the *N.meningitidis* outer membrane protein [106], synthetic peptides [107,108], heat shock proteins [109,110], pertussis proteins [111,112], protein D from *H.influenzae* [113], cytokines [114], lymphokines [114], streptococcal proteins, hormones [114], growth factors [114], toxin A or B from *C.difficile* [115], iron-uptake proteins [116], *etc.* A preferred carrier protein is the CRM197 diphtheria toxoid [117].

Antigens in the composition will typically be present at a concentration of at least 1 µg/ml each. In general, the concentration of any given antigen will be sufficient to elicit an immune response against that antigen.

Immunogenic compositions of the invention may be used therapeutically (*i.e*. to treat an existing infection) or prophylactically (*i.e*. to prevent future infection).

Compositions of the invention may include one, two or three of: (a) saccharide antigens from meningococcus serogroups Y, W135, C and (optionally) A; (b) a saccharide antigen from *Haemophilus influenzae* type B; and/or (c) an antigen from *Streptococcus pneumoniae.*

### Meningococcus serogroups Y. W135, C and (optionally) A

Polysaccharide vaccines against serogroups A, C, W135 & Y have been known for many years. These vaccines (MENCEVAX ACWY™ and MENOMUNE™) are based on the organisms' capsular polysaccharides and, although they are effective in adolescents and adults, they give a poor immune response and short duration of protection, and they cannot be used in infants.

In contrast to the unconjugated polysaccharide antigens in these vaccines, the recently-approved serogroup C vaccines (Menjugate™ [118,85], Meningitec™ and NeisVac-C™) include conjugated saccharides. Menjugate™ and Meningitec™ have oligosaccharide antigens conjugated to a CRM₁₉₇ carrier, whereas NeisVac-C™ uses the complete polysaccharide (de-O-acetylated) conjugated to a tetanus toxoid carrier. The Menactra™ vaccine contains conjugated capsular saccharide antigens from each of serogroups Y, W135, C and A.

Compositions of the present invention preferably include capsular saccharide antigens from one or more of meningococcus serogroups Y, W135, C and (optionally) A, wherein the antigens are conjugated to carrier protein(s) and/or are oligosaccharides. For example, the composition may include a capsular saccharide antigen from: serogroup C; serogroups A and C; serogroups A, C and W135; serogroups A, C and Y; serogroups C, W135 and Y; or from all four of serogroups A, C, W135 and Y.

A typical quantity of each meningococcal saccharide antigen per dose is between 1µg and 20µg *e.g*. about 1µg, about 2.5µg, about 4µg, about 5µg, or about 10µg (expressed as saccharide).

Where a mixture comprises capsular saccharides from both serogroups A and C, the ratio (w/w) of MenA saccharide:MenC saccharide may be greater than 1 (*e.g.* 2:1, 3:1, 4:1, 5:1, 10:1 or higher). Where a mixture comprises capsular saccharides from serogroup Y and one or both of serogroups C and W135, the ratio (w/w) of MenY saccharide:MenW135 saccharide may be greater than 1 (*e.g*. 2:1, 3:1, 4:1, 5:1, 10:1 or higher) and/or that the ratio (w/w) of MenY saccharide:MenC saccharide may be less than 1 (*e.g.* 1:2, 1:3, 1:4, 1:5, or lower). Preferred ratios (w/w) for saccharides from serogroups A:C:W135:Y are: 1:1:1:1; 1:1:1:2; 2:1:1:1; 4:2:1:1; 8:4:2:1; 4:2:1:2; 8:4:1:2; 4:2:2:1; 2:2:1:1; 4:4:2:1; 2:2:1:2; 4:4:1:2; and 2:2:2:1. Preferred ratios (w/w) for saccharides from serogroups C:W135:Y are: 1:1:1; 1:1:2; 1:1:1; 2:1:1; 4:2:1; 2:1:2; 4:1:2; 2:2:1; and 2:1:1. Using a substantially equal mass of each saccharide is preferred.

Capsular saccharides will generally be used in the form of oligosaccharides. These are conveniently formed by fragmentation of purified capsular polysaccharide (*e.g*. by hydrolysis), which will usually be followed by purification of the fragments of the desired size.

Fragmentation of polysaccharides is preferably performed to give a final average degree of polymerisation (DP) in the oligosaccharide of less than 30 (*e.g*. between 10 and 20, preferably around 10 for serogroup A; between 15 and 25 for serogroups W135 and Y, preferably around 15-20; between 12 and 22 for serogroup C; *etc.*). DP can conveniently be measured by ion exchange chromatography or by colorimetric assays [119].

If hydrolysis is performed, the hydrolysate will generally be sized in order to remove short-length oligosaccharides [86]. This can be achieved in various ways, such as ultrafiltration followed by ion-exchange chromatography. Oligosaccharides with a degree of polymerisation of less than or equal to about 6 are preferably removed for serogroup A, and those less than around 4 are preferably removed for serogroups W135 and Y.

Preferred MenC saccharide antigens are disclosed in reference 118, as used in Menjugate™.

The saccharide antigen may be chemically modified. This is particularly useful for reducing hydrolysis for serogroup A [120; see below]. De-O-acetylation of meningococcal saccharides can be performed. For oligosaccharides, modification may take place before or after depolymerisation.

Where a composition of the invention includes a MenA saccharide antigen, the antigen is preferably a modified saccharide in which one or more of the hydroxyl groups on the native saccharide has/have been replaced by a blocking group [120]. This modification improves resistance to hydrolysis.

The number of monosaccharide units having blocking groups can vary. For example, all or substantially all the monosaccharide units may have blocking groups. Alternatively, at least 10%, 20%, 30%, 40%, 50%, 60%, 70%, 80% or 90% of the monosaccharide units may have blocking groups. At least 1, 2, 3, 4, 5, 6, 7, 8, 9, 10, 11, 12, 13, 14, 15, 16, 17, 118, 19, 20, 21, 22, 23, 24, 25, 26, 27, 28, 29 or 30 monosaccharide units may have blocking groups.

Likewise, the number of blocking groups on a monosaccharide unit may vary. For example, the number of blocking groups on a monosaccharide unit may be 1 or 2. The blocking group will generally be at the 4 position and/or 3-position of the monosaccharide units.

The terminal monosaccharide unit may or may not have a blocking group instead of its native hydroxyl. It is preferred to retain a free anomeric hydroxyl group on a terminal monosaccharide unit in order to provide a handle for further reactions (*e.g.* conjugation). Anomeric hydroxyl groups can be converted to amino groups (-NH₂ or NH-E, where E is a nitrogen protecting group) by reductive amination (using, for example, NaBH₃CN/NH₄Cl), and can then be regenerated after other hydroxyl groups have been converted to blocking groups.

Blocking groups to replace hydroxyl groups may be directly accessible via a derivatizing reaction of the hydroxyl group i.e. by replacing the hydrogen atom of the hydroxyl group with another group. Suitable derivatives of hydroxyl groups which act as blocking groups are, for example, carbamates, sulfonates, carbonates, esters, ethers (*e.g.* silyl ethers or alkyl ethers) and acetals. Some specific examples of such blocking groups are allyl, Aloc, benzyl, BOM, t-butyl, trityl, TBS, TBDPS, TES, TMS, TIPS, PMB, MEM, MOM, MTM, THP, *etc.* Other blocking groups that are not directly accessible and which completely replace the hydroxyl group include C₁₋₁₂ alkyl, C₃₋₁₂ alkyl, C₅₋₁₂ aryl, C₅₋₁₂ aryl-C₁₋₆ alkyl, NR¹R² (R¹ and R² are defined in the following paragraph), H, F, Cl, Br, CO₂H, CO₂(C₁₋₆ alkyl), CN, CF₃, CCl₃, *etc*. Preferred blocking groups are electron-withdrawing groups.

Preferred blocking groups are of the formula: -O-X-Y or -OR³ wherein: X is C(O), S(O) or SO₂; Y is C₁₋₁₂ alkyl, C₁₋₁₂ alkoxy, C₃₋₁₂ cycloalkyl, C₅₋₁₂ aryl or C₅₋₁₂ aryl-C₁₋₆ alkyl, each of which may optionally be substituted with 1, 2 or 3 groups independently selected from F, Cl, Br, CO₂H, CO₂(C₁₋₆ alkyl), CN, CF₃ or CCl₃; or Y is NR¹R²; R¹ and R² are independently selected from H, C₁₋₁₂ alkyl, C₃₋₁₂ cycloalkyl, C₅₋₁₂ aryl, C₅₋₁₂ aryl-C₁₋₆ alkyl; or R¹ and R² may be joined to form a C₃₋₁₂ saturated heterocyclic group; R³ is C₁₋₁₂ alkyl or C₃₋₁₂ cycloalkyl, each of which may optionally be substituted with 1, 2 or 3 groups independently selected from F, Cl, Br, CO₂(C₁₋₆ alkyl), CN, CF₃ or CCl₃; or R³ is C₅₋₁₂ aryl or C₅₋₁₂ aryl-C₁₋₆ alkyl, each of which may optionally be substituted with 1, 2, 3, 4 or 5 groups selected from F, Cl, Br, CO₂H CO₂(C₁₋₆ alkyl), CN, CF₃ or CCl₃. When R³ is C₁₋₁₂ alkyl or C₃₋₁₂ cycloalkyl, it is typically substituted with 1, 2 or 3 groups as defined above. When R¹ and R² are joined to form a C₃₋₁₂ saturated heterocyclic group, it is meant that R¹ and R² together with the nitrogen atom form a saturated heterocyclic group containing any number of carbon atoms between 3 and 12 (*e.g*. C₃, C₄, C₅, C₆, C₇, C₈, C₉, C₁₀, C₁₁, C₁₂). The heterocyclic group may contain 1 or 2 heteroatoms (such as N, O or S) other than the nitrogen atom. Examples of C₃₋₁₂ saturated heterocyclic groups are pyrrolidinyl, piperidinyl, morpholinyl, piperazinyl, imidazolidinyl, azetidinyl and aziridinyl.

Blocking groups -O-X-Y and -OR³ can be prepared from -OH groups by standard derivatizing procedures, such as reaction of the hydroxyl group with an acyl halide, alkyl halide, sulfonyl halide, etc. Hence, the oxygen atom in -O-X-Y is preferably the oxygen atom of the hydroxyl group, while the -X-Y group in -O-X-Y preferably replaces the hydrogen atom of the hydroxyl group.

Alternatively, the blocking groups may be accessible via a substitution reaction, such as a Mitsonobu-type substitution. These and other methods of preparing blocking groups from hydroxyl groups are well known.

More preferably, the blocking group is -OC(O)CF₃ [121], or a carbamate group -OC(O)NR¹R², where R¹ and R² are independently selected from C₁₋₆ alkyl. More preferably, R¹ and R² are both methyl *i.e*. the blocking group is -OC(O)NMe₂. Carbamate blocking groups have a stabilizing effect on the glycosidic bond and may be prepared under mild conditions.

Preferred modified MenA saccharides contain *n* monosaccharide units, where at least *h*% of the monosaccharide units do not have -OH groups at both of positions 3 and 4. The value of *h* is 24 or more (*e.g*. 25, 26, 27, 28, 29, 30, 35, 40, 45, 50, 55, 60, 65, 70, 75, 80, 85, 90, 95, 98, 99 or 100) and is preferably 50 or more. The absent -OH groups are preferably blocking groups as defined above.

Other preferred modified MenA saccharides comprise monosaccharide units, wherein at least *s* of the monosaccharide units do not have-OH at the 3 position and do not have -OH at the 4 position. The value of *s* is at least 1 (*e.g*. 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90). The absent -OH groups are preferably blocking groups as defined above.

Suitable modified MenA saccharides for use with the invention have the formula: 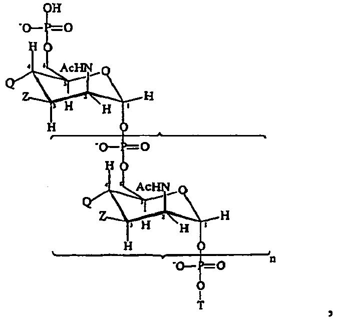 wherein
n is an integer from 1 to 100 (preferably an integer from 15 to 25);
T is of the formula (A) or (B): 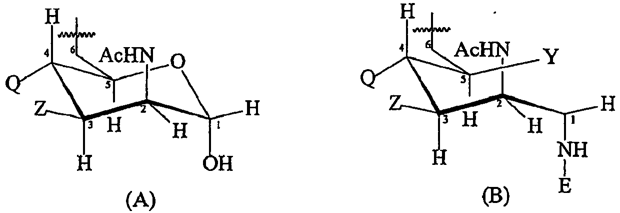 each Z group is independently selected from OH or a blocking group as defined above; and
each Q group is independently selected from OH or a blocking group as defined above;
Y is selected from OH or a blocking group as defined above;
E is H or a nitrogen protecting group;
and wherein more than about 7% (*e.g.* 8%, 9%, 10% or more) of the Q groups are blocking groups.

Each of the *n*+*2* Z groups may be the same or different from each other. Likewise, each of the *n*+*2* Q groups may be the same or different from each other. All the Z groups may be OH. Alternatively, at least 10%, 20, 30%, 40%, 50% or 60% of the Z groups may be OAc. Preferably, about 70% of the Z groups are OAc, with the remainder of the Z groups being OH or blocking groups as defined above. At least about 7% of Q groups are blocking groups. Preferably, at least 10%, 20%, 30%, 40%, 50%, 60%, 70%, 80%, 90% or even 100% of the Q groups are blocking groups.

Meningococcal capsular polysaccharides are typically prepared by a process comprising the steps of polysaccharide precipitation (*e.g*. using a cationic detergent), ethanol fractionation, cold phenol extraction (to remove protein) and ultracentrifugation (to remove LPS) [*e.g*. ref. 122]. A more preferred process [87], however, involves polysaccharide precipitation followed by solubilisation of the precipitated polysaccharide using a lower alcohol. Precipitation can be achieved using a cationic detergent such as tetrabutylammonium and cetyltrimethylammonium salts (*e.g*. the bromide salts), or hexadimethrine bromide and myristyltrimethylammonium salts. Cetyltrimethylammonium bromide ('CTAB') is particularly preferred [123]. Solubilisation of the precipitated material can be achieved using a lower alcohol such as methanol, propan-1-ol, propan-2-ol, butan-1-ol, butan-2-ol, 2-methylpropan-1-ol, 2-methyl-propan-2-ol, diols, *etc*., but ethanol is particularly suitable for solubilising CTAB-polysaccharide complexes. Ethanol is preferably added to the precipitated polysaccharide to give a final concentration (based on total content of ethanol and water) of between 50% and 95%.

After re-solubilisation, the polysaccharide may be further treated to remove contaminants. This is particularly important in situations where even minor contamination is not acceptable (*e.g*. for human vaccine production). This will typically involve one or more steps of filtration *e.g.* depth filtration, filtration through activated carbon may be used, size filtration and/or ultrafiltration. Once filtered to remove contaminants, the polysaccharide may be precipitated for further treatment and/or processing. This can be conveniently achieved by exchanging cations (*e.g.* by the addition of calcium or sodium salts).

As an alternative to purification, capsular saccharides may be obtained by total or partial synthesis *e.g*. Hib synthesis is disclosed in ref. 124, and MenA synthesis in ref. 125.

Compositions of the invention may comprise capsular saccharides from at least two serogroups of *N.meningitidis.* The saccharides are preferably prepared separately (including any fragmentation, conjugation, modification, *etc*.) and then admixed to give a composition of the invention.

Where the composition comprises capsular saccharide from serogroup A, however, it is preferred that the serogroup A saccharide is not combined with the other saccharide(s) until shortly before use, in order to minimise the potential for hydrolysis. This can conveniently be achieved by having the serogroup A component (typically together with appropriate excipients) in lyophilised form and the other serogroup component(s) in liquid form (also with appropriate excipients), with the liquid components being used to reconstitute the lyophilised MenA component when ready for use. Where an aluminium salt adjuvant is used, it is preferred to include the adjuvant in the vial containing the with the liquid vaccine, and to lyophilise the MenA component without adjuvant.

A composition of the invention may thus be prepared from a kit comprising: (a) capsular saccharide from *N.meningitidis* serogroup A, in lyophilised form; and (b) the further antigens from the composition, in liquid form. The invention also provides a method for preparing a composition of the invention, comprising mixing a lyophilised capsular saccharide from *N.meningitidis* serogroup A with the further antigens, wherein said further antigens are in liquid form.

Compositions of the invention can be used in a kit comprising: (a) a first container containing capsular saccharides from two or more of *N.meningitidis* serogroups C, W135 and Y, all in lyophilised form; and (b) a second container containing in liquid form (i) a composition which, after administration to a subject, is able to induce an antibody response in that subject, wherein the antibody response is bactericidal against two or more (*e.g*. 2 or 3) of hypervirulent lineages A4, ET-5 and lineage 3 of *N.meningitidis* serogroup B, (ii) capsular saccharides from none or one of *N.meningitidis* serogroups C, W135 and Y, and optionally (iii) further antigens (see below) that do not include meningococcal capsular saccharides, wherein, reconstitution of the contents of container (a) by the contents of container (b) provides a composition of the invention.

Within each dose, the amount of an individual saccharide antigen will generally be between 1-50 µg (measured as mass of saccharide), with about 2.5µg, 5µg or 10 µg of each being preferred. With A:C:W135:Y weight ratios of 1:1:1:1; 1:1:1:2; 2:1:1:1; 4:2:1:1; 8:4:2:1; 4:2:1:2; 8:4:1:2; 4:2:2:1; 2:2:1:1; 4:4:2:1; 2:2:1:2; 4:4:1:2; and 2:2:2:1, therefore, the amount represented by the number 1 is preferably about 2.5µg, 5µg or 10 µg. For a 1:1:1:1 ratio A:C:W:Y composition and a 10µg per saccharide, therefore, 40 µg saccharide is administered per dose. Preferred compositions have about the following µg saccharide per dose:

| | | | | | | | |
|---|---|---|---|---|---|---|---|
| **A** | 10 | 0 | 0 | 0 | 10 | 5 | 2.5 |
| **C** | 10 | 10 | 5 | 2.5 | 5 | 5 | 2.5 |
| **W135** | 10 | 10 | 5 | 2.5 | 5 | 5 | 2.5 |
| **Y** | 10 | 10 | 5 | 2.5 | 5 | 5 | 2.5 |

Compositions of the invention may comprise less than 50 µg meningococcal saccharide per dose. Other compositions comprise ≤ 40 µg meningococcal saccharide per dose. Other preferred compositions comprise ≤30 µg meningococcal saccharide per dose. Other preferred compositions comprise ≤25 µg meningococcal saccharide per dose. Other preferred compositions comprise ≤20 µg meningococcal saccharide per dose. Other preferred compositions comprise ≤10 µg meningococcal saccharide per dose but, ideally, compositions comprise at least 10 µg meningococcal saccharide per dose.

The Menjugate™ and NeisVac™ MenC conjugates use a hydroxide adjuvant, whereas Meningitec™ uses a phosphate. It is possible in compositions of the invention to adsorb some antigens to an aluminium hydroxide but to have other antigens in association with an aluminium phosphate. For tetravalent serogroup combinations, for example, the following permutations are available:

| **Serogroup** | **Aluminium salt** (H = a hydroxide; P = a phosphate) | | | | | | | | | | | | | | | |
|---|---|---|---|---|---|---|---|---|---|---|---|---|---|---|---|---|
| A | P | H | P | H | H | H | P | P | P | H | H | H | P | P | P | H |
| C | P | H | H | P | H | H | P | H | H | P | P | H | P | H | P | P |
| W135 | P | H | H | H | P | H | H | P | H | H | P | P | P | P | H | P |
| Y | P | H | H | H | H | P | H | H | H | P | H | P | H | P | P | P |

For trivalent *N.meningitidis* serogroup combinations, the following permutations are available:

| **Serogroup** | **Aluminium salt** (H = a hydroxide; P = a phosphate) | | | | | | | |
|---|---|---|---|---|---|---|---|---|
| C | P | H | H | H | P | P | P | H |
| W135 | P | H | H | P | H | P | H | P |
| Y | P | H | P | H | H | H | P | P |

### Haemophilus influenzae type B

Where the composition includes a *H.influenzae* type B antigen, it will typically be a Hib capsular saccharide antigen. Saccharide antigens from *H.influenzae* b are well known.

Advantageously, the Hib saccharide is covalently conjugated to a carrier protein, in order to enhance its immunogenicity, especially in children. The preparation of polysaccharide conjugates in general, and of the Hib capsular polysaccharide in particular, is well documented [*e.g*. references 126 to 134 *etc*.]. The invention may use any suitable Hib conjugate. Suitable carrier proteins are described below, and preferred carriers for Hib saccharides are CRM₁₉₇ ('HbOC'), tetanus toxoid ('PRP-T') and the outer membrane complex of *N.meningitidis* ('PRP-OMP').

The saccharide moiety of the conjugate may be a polysaccharide (*e.g.* full-length polyribosylribitol phosphate (PRP)), but it is preferred to hydrolyse polysaccharides to form oligosaccharides (*e.g.* MW from ~1 to ~5 kDa).

A preferred conjugate comprises a Hib oligosaccharide covalently linked to CRM₁₉₇ via an adipic acid linker [135, 136]. Tetanus toxoid is also a preferred carrier.

Compositions of the invention may comprise more than one Hib antigen.

Where a composition includes a Hib saccharide antigen, it is preferred that it does not also include an aluminium hydroxide adjuvant. If the composition includes an aluminium phosphate adjuvant then the Hib antigen may be adsorbed to the adjuvant [137] or it may be non-adsorbed [138].

Hib antigens may be lyophilised *e.g.* together with meningococcal antigens.

### Streptococcus pneumoniae

Where the composition includes a *S.pneumoniae* antigen, it will typically be a capsular saccharide antigen which is preferably conjugated to a carrier protein [*e.g.* refs. 88-90]. It is preferred to include saccharides from more than one serotype of *S.pneumoniae.* For example, mixtures of polysaccharides from 23 different serotype are widely used, as are conjugate vaccines with polysaccharides from between 5 and 11 different serotypes [139]. For example, PrevNar™ [140] contains antigens from seven serotypes (4, 6B, 9V, 14, 18C, 19F, and 23F) with each saccharide individually conjugated to CRM₁₉₇ by reductive amination, with 2µg of each saccharide per 0.5ml dose (4µg of serotype 6B), and with conjugates adsorbed on an aluminium phosphate adjuvant. Compositions of the invention preferably include at least serotypes 6B, 14, 19F and 23F. Conjugates may be adsorbed onto an aluminium phosphate.

As an alternative to using saccharide antigens from pneumococcus, the composition may include one or more polypeptide antigens. Genome sequences for several strains of pneumococcus are available [141,142] and can be subjected to reverse vaccinology [143-146] to identify suitable polypeptide antigens [147,148]. For example, the composition may include one or more of the following antigens: PhtA, PhtD, PhtB, PhtE, SpsA, LytB, LytC, LytA, Sp125, Sp101, Sp128 and Sp130, as defined in reference 149.

In some embodiments, the composition may include both saccharide and polypeptide antigens from pneumococcus. These may be used in simple admixture, or the pneumococcal saccharide antigen may be conjugated to a pneumococcal protein. Suitable carrier proteins for such embodiments include the antigens listed in the previous paragraph [149].

Pneumococcal antigens may be lyophilised *e.g*. together with meningococcal and/or Hib antigens.

### Covalent conjugation

Capsular saccharides in compositions of the invention will usually be conjugated to carrier protein(s). In general, conjugation enhances the immunogenicity of saccharides as it converts them from T-independent antigens to T-dependent antigens, thus allowing priming for immunological memory. Conjugation is particularly useful for paediatric vaccines and is a well known technique [*e.g*. reviewed in refs. 150 and 126-134].

Preferred carrier proteins are bacterial toxins or toxoids, such as diphtheria toxoid or tetanus toxoid. The CRM₁₉₇ mutant diphtheria toxin [117,151,152] is particularly preferred. Other suitable carrier proteins include the *N.meningitidis* outer membrane protein [106], synthetic peptides [107,108], heat shock proteins [109,110], pertussis proteins [111,112], cytokines [114], lymphokines [114], hormones [114], growth factors [114], artificial proteins comprising multiple human CD4⁺ T cell epitopes from various pathogen-derived antigens [153], protein D from *H.influenzae* [113,154], pneumococcal surface protein PspA [155], iron-uptake proteins [116], toxin A or B from *C.difficile* [115], *etc.* Preferred carriers are diphtheria toxoid, tetanus toxoid, *H.influenzae* protein D, and CRM₁₉₇.

Within a composition of the invention, it is possible to use more than one carrier protein *e.g*. to reduce the risk of carrier suppression. Thus different carrier proteins can be used for different serogroups *e.g*. serogroup A saccharides might be conjugated to CRM₁₉₇ while serogroup C saccharides might be conjugated to tetanus toxoid. It is also possible to use more than one carrier protein for a particular saccharide antigen *e.g*. serogroup A saccharides might be in two groups, with some conjugated to CRM₁₉₇ and others conjugated to tetanus toxoid. In general, however, it is preferred to use the same carrier protein for all saccharides.

A single carrier protein might carry more than one saccharide antigen [156]. For example, a single carrier protein might have conjugated to it saccharides from serogroups A and C. To achieve this goal, saccharides can be mixed prior to the conjugation reaction. In general, however, it is preferred to have separate conjugates for each serogroup.

Conjugates with a saccharide:protein ratio (w/w) of between 1:5 (*i.e*. excess protein) and 5:1 (*i.e.* excess saccharide) are preferred. Ratios between 1:2 and 5:1 are preferred, as are ratios between 1:1.25 and 1:2.5 are more preferred. Excess carrier protein is preferred for MenA and MenC.

Conjugates may be used in conjunction with free carrier protein [157]. When a given carrier protein is present in both free and conjugated form in a composition of the invention, the unconjugated form is preferably no more than 5% of the total amount of the carrier protein in the composition as a whole, and more preferably present at less than 2% by weight.

Any suitable conjugation reaction can be used, with any suitable linker where necessary.

The saccharide will typically be activated or functionalised prior to conjugation. Activation may involve, for example, cyanylating reagents such as CDAP (*e.g*. 1-cyano-4-dimethylamino pyridinium tetrafluoroborate [158,159,*etc*.]). Other suitable techniques use carbodiimides, hydrazides, active esters, norborane, p-nitrobenzoic acid, N-hydroxysuccinimide, S-NHS, EDC, TSTU; see also the introduction to reference 132).

Linkages via a linker group may be made using any known procedure, for example, the procedures described in references 160 and 161. One type of linkage involves reductive amination of the polysaccharide, coupling the resulting amino group with one end of an adipic acid linker group, and then coupling a protein to the other end of the adipic acid linker group [130,162,163]. Other linkers include B-propionamido [164], nitrophenyl-ethylamine [165], haloacyl halides [166], glycosidic linkages [167], 6-aminocaproic acid [168], ADH [169], C₄ to C₁₂ moieties [170] *etc.* As an alternative to using a linker, direct linkage can be used. Direct linkages to the protein may comprise oxidation of the polysaccharide followed by reductive amination with the protein, as described in, for example, references 171 and 172.

A process involving the introduction of amino groups into the saccharide (*e.g*. by replacing terminal =O groups with -NH₂) followed by derivatisation with an adipic diester (*e.g.* adipic acid N-hydroxysuccinimido diester) and reaction with carrier protein is preferred. Another preferred reaction uses CDAP activation with a protein D carrier *e.g*. for MenA or MenC.

After conjugation, free and conjugated saccharides can be separated. There are many suitable methods, including hydrophobic chromatography, tangential ultrafiltration, diafiltration *etc.* [see also refs. 173 & 174, *etc*.].

Where the composition of the invention includes a conjugated oligosaccharide, it is preferred that oligosaccharide preparation precedes conjugation.

### Outer membrane vesicles

It is preferred that compositions of the invention should not include complex or undefined mixtures of antigens, which are typical characteristics of OMVs. However, one way in which the invention can be applied to OMVs is where OMVs are to be administered in a multiple dose schedule.

Where more than one OMV dose is to be administered, each dose may be supplemented (either by adding the purified protein or by expression of the protein within the bacteria from which the OMVs are derived) by one of the first protein, second protein or third protein as defined above. Preferably different doses are supplemented with different NMB1870 families. In a three dose OMV schedule, for example, each dose could contain a different one of the first protein, second protein and third protein such that, after receiving three doses of OMVs, all three families have been received. In a two dose OMV schedule, one family could be used per OMV dose (thus omitting one family), or one or both OMV doses could be supplemented with more than one family in order to give coverage with all three families. In preferred embodiments, there are three OMV doses, and each of the three OMV doses contains three different genetically-engineered vesicle populations each displaying three subtypes, thereby giving nine different subtypes in all.

This approach may be used in general to improve preparations of *N.meningitidis* serogroup B microvesicles [175], 'native OMVs' [176], blebs or outer membrane vesicles [*e.g*. refs. 177 to 182, *etc*.]. These may be prepared from bacteria which have been genetically manipulated [183-186] *e.g.* to increase immunogenicity (*e.g*. hyper-express immunogens), to reduce toxicity, to inhibit capsular polysaccharide synthesis, to down-regulate PorA expression, *etc.* They may be prepared from hyperblebbing strains [187-190]. Vesicles from a non-pathogenic *Neisseria* may be included [191]. OMVs may be prepared without the use of detergents [192,193]. They may express non-Neisserial proteins on their surface [194]. They may be LPS-depleted. They may be mixed with recombinant antigens [177,195]. Vesicles from bacteria with different class I outer membrane protein subtypes may be used e.g. six different subtypes [196,197] using two different genetically-engineered vesicle populations each displaying three subtypes, or nine different subtypes using three different genetically-engineered vesicle populations each displaying three subtypes, etc. Useful subtypes include: P1.7,16; P1.5-1,2-2; P1.19,15-1; P1.5-2,10; P1.12-1,13; P1.7-2,4; P1.22,14; P41.7-1,1; P1.18-1,3,6.

It is also possible, of course, to supplement vesicle preparations with two or three different families.

### Protein expression

Bacterial expression techniques are known in the art. A bacterial promoter is any DNA sequence capable of binding bacterial RNA polymerase and initiating the downstream (3') transcription of a coding sequence (*e.g*. structural gene) into mRNA. A promoter will have a transcription initiation region which is usually placed proximal to the 5' end of the coding sequence. This transcription initiation region usually includes an RNA polymerase binding site and a transcription initiation site. A bacterial promoter may also have a second domain called an operator, that may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the lac operon in Escherichia coli (E. coli) [Raibaud et al. (1984) Annu. Rev. Genet. 18:173]. Regulated expression may therefore be either positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose (*lac*) [Chang et al. (1977) Nature 198:1056*],* and maltose. Additional examples include promoter sequences derived from biosynthetic enzymes such as tryptophan (*trp*) [Goeddel et al. (1980)Nuc. Acids Res. 8:4057; Yelverton et al. (1981) Nucl. Acids Res. 9:731; US patent 4,738,921; EP-A-0036776 and EP-A-0121775]. The β-lactamase (*bla*) promoter system [Weissmann (1981) "The cloning of interferon and other mistakes." In Interferon 3 (ed. I. Gresser)], bacteriophage lambda PL [Shimatake et al. (1981) Nature 292:128] and T5 [US patent 4,689,406] promoter systems also provide useful promoter sequences. Another promoter of interest is an inducible arabinose promoter (pBAD).

In addition, synthetic promoters which do not occur in nature also function as bacterial promoters. For example, transcription activation sequences of one bacterial or bacteriophage promoter may be joined with the operon sequences of another bacterial or bacteriophage promoter, creating a synthetic hybrid promoter [US patent 4,551,433]. For example, the *tac* promoter is a hybrid *trp-lac* promoter comprised of both *trp* promoter and *lac* operon sequences that is regulated by the *lac* repressor [Amann et al. (1983) Gene 25:167; de Boer et al. (1983) Proc. Natl. Acad Sci 80:21]*.* Furthermore, a bacterial promoter can include naturally occurring promoters of non-bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophage T7 RNA polymerase/promoter system is an example of a coupled promoter system. [Studier et al. (1986) J. Mol. Biol. 189:113; Tabor et al. (1985) Proc Natl. Acad. Sci. 82:1074]. In addition, a hybrid promoter can also be comprised of a bacteriophage promoter and an *E*. *coli* operator region (EPO-A-0 267 851).

In addition to a functioning promoter sequence, an efficient ribosome binding site is also useful for the expression of foreign genes in prokaryotes. In *E*. *coli,* the ribosome binding site is called the Shine-Dalgamo (SD) sequence and includes an initiation codon (ATG) and a sequence 3-9 nucleotides in length located 3-11 nucleotides upstream of the initiation codon [Shine et al. (1975) Nature 254:34]. The SD sequence is thought to promote binding of mRNA to the ribosome by the pairing of bases between the SD sequence and the 3' and of *E*. *coli* 16S rRNA [Steitz et al. (1979) "Genetic signals and nucleotide sequences in messenger RNA." In Biological Regulation and Development. Gene Expression (ed. R.F. Goldberger)]. To express eukaryotic genes and prokaryotic genes with weak ribosome-binding site [Sambrook et al. (1989) "Expression of cloned genes in Escherichia coli." In Molecular Cloning: A Laboratory Manual].

A promoter sequence may be directly linked with the DNA molecule, in which case the first amino acid at the N-terminus will always be a methionine, which is encoded by the ATG start codon. If desired, methionine at the N-terminus may be cleaved from the protein by *in vitro* incubation with cyanogen bromide or by either *in vivo* on *in vitro* incubation with a bacterial methionine N-terminal peptidase (EP-A-0219237).

Usually, transcription termination sequences recognized by bacteria are regulatory regions located 3' to the translation stop codon, and thus together with the promoter flank the coding sequence. These sequences direct the transcription of an mRNA which can be translated into the polypeptide encoded by the DNA. Transcription termination sequences frequently include DNA sequences of about 50 nucleotides capable of forming stem loop structures that aid in terminating transcription. Examples include transcription termination sequences derived from genes with strong promoters, such as the *trp* gene in *E*. *coli* as well as other biosynthetic genes.

Usually, the above described components, comprising a promoter, signal sequence (if desired), coding sequence of interest, and transcription termination sequence, are put together into expression constructs. Expression constructs are often maintained in a replicon, such as an extrachromosomal element (*e.g.* plasmids) capable of stable maintenance in a host, such as bacteria. The replicon will have a replication system, thus allowing it to be maintained in a prokaryotic host either for expression or for cloning and amplification. In addition, a replicon may be either a high or low copy number plasmid. A high copy number plasmid will generally have a copy number ranging from about 5 to about 200, and usually about 10 to about 150. A host containing a high copy number plasmid will preferably contain at least about 10, and more preferably at least about 20 plasmids. Either a high or low copy number vector may be selected, depending upon the effect of the vector and the foreign protein on the host.

Alternatively, the expression constructs can be integrated into the bacterial genome with an integrating vector. Integrating vectors usually contain at least one sequence homologous to the bacterial chromosome that allows the vector to integrate. Integrations appear to result from recombinations between homologous DNA in the vector and the bacterial chromosome. For example, integrating vectors constructed with DNA from various Bacillus strains integrate into the Bacillus chromosome (EP-A-0127328). Integrating vectors may also be comprised of bacteriophage or transposon sequences.

Usually, extrachromosomal and integrating expression constructs may contain selectable markers to allow for the selection of bacterial strains that have been transformed. Selectable markers can be expressed in the bacterial host and may include genes which render bacteria resistant to drugs such as ampicillin, chloramphenicol, erythromycin, kanamycin (neomycin), and tetracycline [Davies et al. (1978) Annu. Rev. Microbiol. 32:469]. Selectable markers may also include biosynthetic genes, such as those in the histidine, tryptophan, and leucine biosynthetic pathways.

Alternatively, some of the above described components can be put together in transformation vectors. Transformation vectors are usually comprised of a selectable market that is either maintained in a replicon or developed into an integrating vector, as described above.

Expression and transformation vectors, either extra-chromosomal replicons or integrating vectors, have been developed for transformation into many bacteria. For example, expression vectors have been developed for, *inter alia*, the following bacteria: Bacillus subtilis [Palva et al. (1982) Proc. Natl. Acad. Sci. USA 79:5582; EP-A-0 036 259 and EP-A-0 063 953; WO 84/04541], Escherichia coli [Shimatake et al. (1981) Nature 292:128; Amann et al. (1985) Gene 40:183; Studier et al. (1986) J. Mol. Biol. 189:113; EP-A-0 036 776,EP-A-0 136 829 and EP-A-0 136 907], Streptococcus cremoris [Powell et al. (1988) Appl. Environ. Microbiol. 54:655]; Streptococcus lividans [Powell et al. (1988) Appl. Environ. Microbiol. 54:655], Streptomyces lividans [US patent 4,745,056].

Methods of introducing exogenous DNA into bacterial hosts are well-known in the art, and usually include either the transformation of bacteria treated with CaCl₂ or other agents, such as divalent cations and DMSO. DNA can also be introduced into bacterial cells by electroporation. Transformation procedures usually vary with the bacterial species to be transformed. See *e.g*. [Masson et al. (1989) FEMSMicrobiol. Lett. 60:273; Palva et al. (1982) Proc. Natl. Acad. Sci. USA 79:5582; EP-A-0 036 259 and EP-A-0 063 953; WO 84/04541, Bacillus], [Miller et al. (1988) Proc. Natl. Acad. Sci. 85:856; Wang et al. (1990) J. Bacteriol. 172:949, Campylobacter], [Cohen et al. (1973) Proc. Natl. Acad. Sci. 69:2110; Dower et al. (1988) Nucleic Acids Res. 16:6127; Kushner (1978) "An improved method for transformation of Escherichia coli with ColEl-derived plasmids. In Genetic Engineering: Proceedings of the International Symposium on Genetic Engineering (eds. H.W. Boyer and S. Nicosia); Mandel et al. (1970) J. Mol. Biol. 53:159; Taketo (1988) Biochim. Biophys. Acta 949:318; Escherichia], [Chassy et al. (1987) FEMS Microbiol. Lett. 44:173 Lactobacillus]; [Fiedler et al. (1988) Anal. Biochem 170:38, Pseudomonas]; [Augustin et al. (1990) FEMS Microbial. Lett. 66:203, Staphylococcus], [Barany et al. (1980) J. Bacteriol. 144:698; Harlander (1987) "Transformation of Streptococcus lactis by electroporation, in: Streptococcal Genetics (ed. J. Ferretti and R. Curtiss III); Perry et al. (1981) Infect. Immun. 32:1295; Powell et al. (1988) Appl. Errviron. Microbiol. 54:655; Somkuti et al. (1987) Proc. 4th Evr. Cong. Biotechnology 1:412, Streptococcus].

### General

The term "comprising" encompasses "including" as well as "consisting" *e.g*. a composition "comprising" X may consist exclusively of X or may include something additional *e.g*. X + Y.

The term "about" in relation to a numerical value *x* means, for example, *x*±10%.

The word "substantially" does not exclude "completely" *e.g*. a composition which is "substantially free" from Y may be completely free from Y. Where necessary, the word "substantially" may be omitted from the definition of the invention.

"Sequence identity" is preferably determined by the Smith-Waterman homology search algorithm as implemented in the MPSRCH program (Oxford Molecular), using an affine gap search with parameters *gap open penalty=12* and *gap extension penalty=1.*

After serogroup, meningococcal classification includes serotype, serosubtype and then immunotype, and the standard nomenclature lists serogroup, serotype, serosubtype, and immunotype, each separated by a colon e.g. B:4:P1.15:L3,7,9. Within serogroup B, some lineages cause disease often (hyperinvasive), some lineages cause more severe forms of disease than others (hypervirulent), and others rarely cause disease at all. Seven hypervirulent lineages are recognised, namely subgroups I, III and IV-1, ET-5 complex, ET-37 complex, A4 cluster and lineage 3. These have been defined by multilocus enzyme electrophoresis (MLEE), but multilocus sequence typing (MLST) has also been used to classify meningococci [ref. 10]. The four main hypervirulent clusters are ST32, ST44, ST8 and ST11 complexes.

The term "alkyl" refers to alkyl groups in both straight and branched forms, The alkyl group may be interrupted with 1, 2 or 3 heteroatoms selected from -O-, -NH- or -S-. The alkyl group may also be interrupted with 1, 2 or 3 double and/or triple bonds. However, the term "alkyl" usually refers to alkyl groups having no heteroatom interruptions or double or triple bond interruptions. Where reference is made to C₁₋₁₂ alkyl, it is meant the alkyl group may contain any number of carbon atoms between 1 and 12 (*e.g.* C₁, C₂, C₃, C₄, C₅, C₆, C₇, C₈, C₉, C₁₀, C_{11,} C₁₂). Similarly, where reference is made to C₁₋₆ alkyl, it is meant the alkyl group may contain any number of carbon atoms between 1 and 6 (e.g. C₁, C₂, C₃, C₄, C₅, C₆).

The term "cycloalkyl" includes cycloalkyl, polycycloalkyl, and cycloalkenyl groups, as well as combinations of these with alkyl groups, such as cycloalkylalkyl groups. The cycloalkyl group may be interrupted with 1, 2 or 3 heteroatoms selected from -O-, -NH- or -S-. However, the term "cycloalkyl" usually refers to cycloalkyl groups having no heteroatom interruptions Examples of cycloalkyl groups include cyclopentyl, cyclohexyl, cyclohexenyl, cyclohexylmethyl and adamantyl groups. Where reference is made to C₃₋₁₂ cycloalkyl, it is meant that the cycloalkyl group may contain any number of carbon atoms between 3 and 12 (*e.g*. C₃, C₄, C₅, C₆, C₇, C₈, C₉, C₁₀, C₁₁, C₁₂).

The term "aryl" refers to an aromatic group, such as phenyl or naphthyl. Where reference is made to C₅₋₁₂ aryl, it is meant that the aryl group may contain any number of carbon atoms between 5 and 12 (*e.g*. C₅, C₆, C₇, C₈, C₉, C₁₀, C₁₁, C₁₂).

The term "C₅₋₁₂ aryl-C₁₋₆ alkyl," refers to groups such as benzyl, phenylethyl and naphthylmethyl.

Nitrogen protecting groups include silyl groups (such as TMS, TES, TBS, TIPS), acyl derivatives (such as phthalimides, trifluoroacetamides, methoxycarbonyl, ethoxycarbonyl, t-butoxycarbonyl (Boc), benzyloxycarbonyl (Z or Cbz), 9-fluorenylmethoxycarbonyl (Fmoc), 2-(trimethylsilyl)ethoxy carbonyl, 2,2,2-trichloroethoxycarbonyl (Troc)), sulfonyl derivatives (such as β-trimethylsilylethanesulfonyl (SES)), sulfenyl derivatives, C₁₋₁₂ alkyl, benzyl, benzhydryl, trityl, 9-phenylfluorenyl, *etc.* A preferred nitrogen protecting group is Fmoc.

In general, the invention does not encompass the various NMB1870 sequences specifically disclosed in references 3, 5, 6 and 7, although these NMB1870 sequences may be used according to the invention e.g, for the construction of chimeric sequences, *etc*.

### BRIEF DESCRIPTION OF DRAWINGS

Figures 1 to 6 show 3D models of NMB1870. Figure 7 shows surface loop transfer for NMB1870.
Figure 8 shows 12mer PepScan epitope mapping of a family I NMB1870 protein. The 3 panels from top to bottom are the results using antisera generated against NMB1870 families I, II and III. Results are in arbitrary dye units.
Figure 9 shows a western blot of fragments of NMB1870, stained using polyclonal serum.
Figure 10 shows FACS analysis using antisera raised against different NMB1870 fragments.
Figure 11 shows a western blot of strains in NMB1870 family I, II or III, stained either with monoclonal antibody mAb502 (left blot) or with a polyclonal anti-NMB1870 serum (right blot).

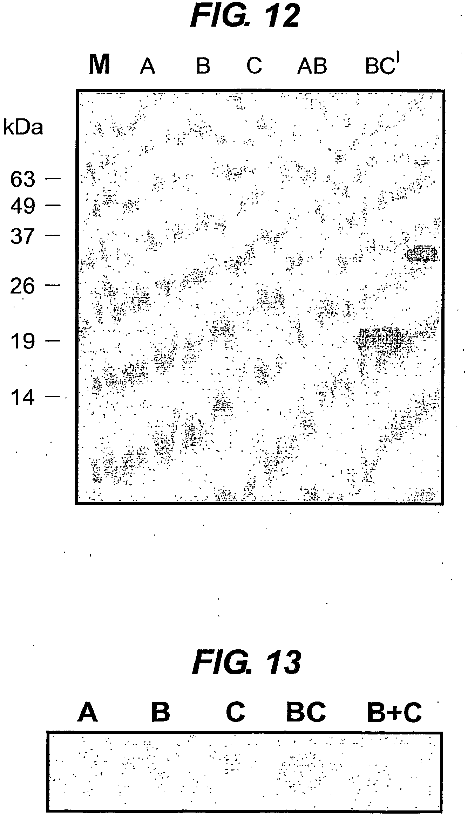

Figure 12 shows a western blot of fragments of NMB1870, stained using mAb502.
Figure 13 shows a dot blot of fragments of NMB1870, stained using mAb502. Domains A to C were tested individually. A domain B-C fragment was also tested, as was a mixture of domains B & C.
Figures 14 and 15 show FACS analysis of bacteria. The three rows were stained with different antibodies: top = mAb502; middle = polyclonal serum; bottom = monoclonal antibody against capsular saccharide (positive control, SEAM3). The three columns in Figure 14 are all family I strains: MC58, M2934 and BZ83. The three columns in Figure 15 do not express family I NMB1870: ΔNMB1870 isogenic knockout of strain MC58; family II strain 961-5945; family III strain M1239.
Figure 16 shows hydrophilicity and secondary structure analyses of the NMB1870_{MC58} sequence (SEQ ID NO: 1) from residues 120 to 274.

### MODES FOR CARRYING OUT THE INVENTION

### Epitope mapping

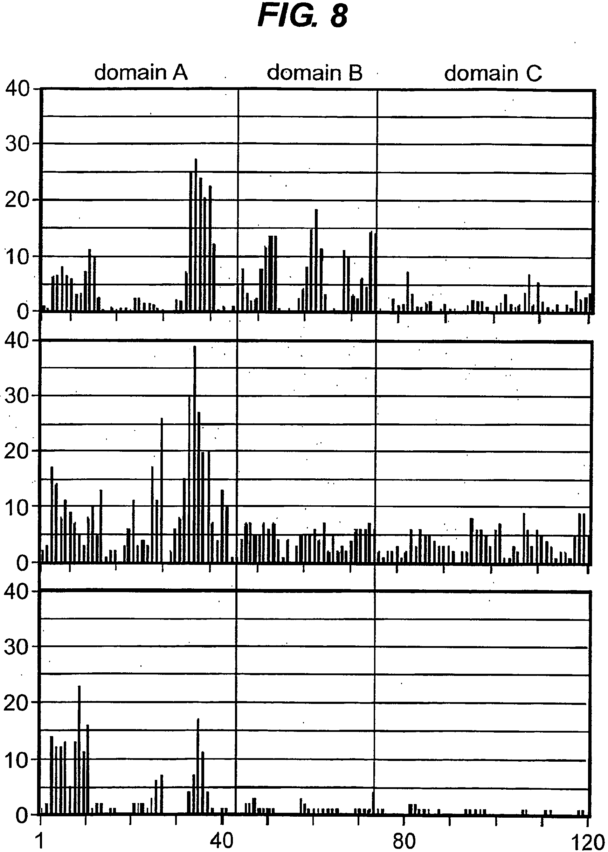

12mer and 10mer fragments of NMB1870_{MC58} were used for 'PepScan' epitope mapping. The fragments were immobilised on a cellulose membrane and reacted with antisera raised against one strain from each of the three NMB1870 families: (I) MC58; (II) 2996; and (III) M1239. The results of the 12mer analysis are shown in Figure 8.

The region including approximately the first 110 amino acids of NMB1870 contains linear epitopes that are common to the three families. Residues 120-183 includes family-specific epitopes. No positive peptides were seen further downstream, suggesting either that these sequences are buried in the protein's 3D structure and so could not elicit any antibodies in the serum, or that the epitopes here are discontinuous and are not seen in the 'PepScan' analysis. The following alignment shows the regions of the NMB1870_{MC58} sequence (SEQ ID NO: 1) which reacted with each antiserum:

| | |
|---|---|
| (1) Anti-MC58 | MNRTAFCCLSLTTALILTACSSGGGGVAADIGAGLADALTAPLDHK**DKGL** |
| (2) Anti-2996 | MNRTAFCCLSLTTALILTACSSGGGGVAAD**IGAGLADALTAPLDHKDKGL** |
| (3) Anti-M1239 | MNRTAFCCLSLTTALI**LTACSSGGGGVAADIGAGLADALTAPLDHKDKGL** |
| | |
| (1) Anti-MC58 | **QSLTLDQSVR**KNEKLKLAAQGAEKTYGNGDSLNTGKLKND**KVSRFDFIRQ** |
| (2) Anti-2996 | **QSLTLDQSVRKNEK**LK**LAAQGAEKTYGNGDSLNTGKLKNDKVSRFDFIRQ** |
| (3) Anti-M1239 | **QSLTLDQS**VRKNEKLKLAAQGAEKTYGNGDSLNTGKLKNDKVSR**FDFIRQ** |
| | |
| (1) Anti-MC58 | **IEVDGQLITLES**GEFQVYKQSH**SALTAFQTEQIQDSEHS**GKM**VAKRQFRI** |
| (2) Anti-2996 | **IEVDGQLITL**ESGEFQVYKQSHSALTAFQTEQIQDSEHSGKMVAKRQFRI |
| (3) Anti-M1239 | **IEVDGQLI**TLESGEFQVYKQSHSALTAFQTEQIQDSEHSGKMVAKRQFRI |
| | |
| (1) Anti-MC58 | **GDIAGEHTSFDKLPEGGRATYRGTAFGSDDAG**GKLTYTIDFAAKQGNGKI |
| (2) Anti-2996 | GDIAGEHTSFDKLPEGGRATYRGTAFGSDDAGGKLTYTIDFAAKQGNGKI |
| (3) Anti-M1239 | GDIAGEHTSFDKLPEGGRATYRGTAFGSDDAGGKLTYTIDFAAKQGNGKI |
| | |
| (1) Anti-MC58 | EHLKSPELNVDLAAADIKPDGKRHAVISGSVLYNQAEKGSYSLGIFGGKA |
| (2) Anti-2996 | EHLKSPELNVDLAAADIKPDGKRHAVISGSVLYNQAEKGSYSLGIFGGKA |
| (3) Anti-M1239 | EHLKSPELNVDLAAADIKPDGKRHAVISGSVLYNQAEKGSYSLGIFGGKA |
| | |
| (1) Anti-MC58 | QEVAGSAEVKTVNGIRHIGLAAKQ |
| (2) Anti-2996 | QEVAGSAEVKTVNGIRHIGLAAKQ |
| (3) Anti-M1239 | QEVAGSAEVKTVNGIRHIGLAAKQ |

The common epitopes for all three families are thus DKGLQSLTLDQSVR (SEQ ID NO: 21) and FDFIRQIEVDGQLI (SEQ ID NO: 22).

Based on the epitope mapping results, the NMB1870 sequence was split into three notional domains:
**(A) Amino acids 1-119** (SEQ ID NO: 4) 
MNRTAFCCLSLTTALILTACSSGGGGVAADIGAGLADALTAPLDHKDKGLQSLTLDQSVRKNEKL
KLAAQGAEKTYGNGDSLNTGKLKNDKVSRFDFIRQIEVDGQLITLESGEFQVYK
**(B) Amino acids 120-184** (SEQ ID NO: 5)
   QSHSALTAFQTEQIQDSEHSGKMVAKRQFRIGDIAGEHTSFDKLPEGGRATYRGTAFGSDDAGG
**(C) Amino acids 185-274** (SEQ ID NO: 6)

KLTYTIDFAAKQGNGKIEHLKSPELNVDLAAADIKPDGKRHAVISGSVLYNQAEKGSYSLGIFGG
KAQEVAGSAEVKTVNGIRHIGLAAKQ

These domains were expressed individually as proteins comprising domains A, B or C (SEQ ID NOS: 4 to 6), and together as proteins comprising A-B (SEQ ID NO: 23) or B-C (SEQ ID NO: 24).

The following oligonucleotide primers were using during the construction of these proteins, and introduce *Nde*I & *Xho*I restriction sites:

| **Protein** | **Primer SEQ ID NOS** (Fwd & Rev) |
|---|---|
| (A) | 25 & 26 |
| (B) | 27 & 28 |
| (C) | 29 & 30 |
| (A)(B) | 31 & 32 |
| (B)(C) | 33 & 34 |

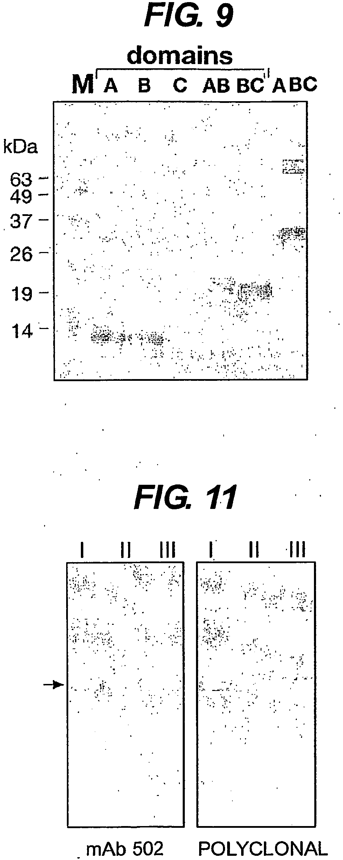

A western blot of domains A (truncated to start with VAA...), B and C, using polyclonal anti-NMB1870_{MC58} as the label, are shown in Figure 9. Fusions AB and BC were also included. The final lane of the blot contains the full-length protein.

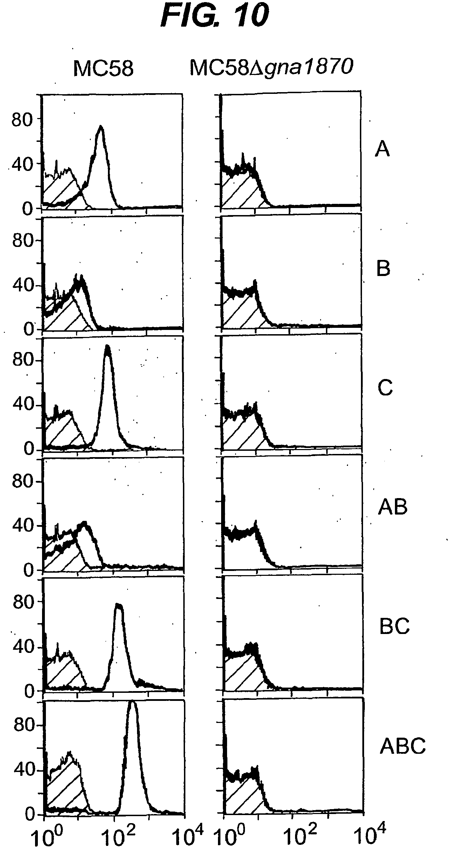

Antisera against each of domains A, B and C were able to bind to whole cells in FACS analysis (Figure 10). The fluorescence shift was strongest with domains A and C, suggesting that they may be more immunoaccessible. None of the antisera could recognise a ΔNMB1870 knockout strain.

Sera were raised in mice against the proteins (and against control proteins) using either CFA or an aluminium hydroxide (AH) as adjuvant, and SBA results against three different meningococcal strains were as follows:

| | **MC58^{FAMILY I}**B:15:P1.7,16b (ET5) | | **961-5945 ^{FAMILY II}**B:2b:P1.21,16 (A4) | | **M1239 ^{FAMILY III}**B:14:P123,14 (lin.3) | |
|---|---|---|---|---|---|---|
| **Protein** | **CFA** | **AH** | **CFA** | **AH** | **CFA** | **AH** |
| | | | | | | |
| A | <4 | 16 | <4 | <4 | <4 | <4 |
| B | <4 | <4 | <4 | <4 | <4 | 256 |
| C | <4 | 512 | <4 | <4 | <4 | <4 |
| AB | <4 | <4 | 256 | 256 | <4 | <4 |
| BC | 32768 | 8192 | <4 | <4 | <4 | <4 |
| | | | | | | |
| SEQ ID NO: 1 | 524288 | 16384 | 2048 | <8 | <4 | <4 |
| SEQ ID NO: 2 | <4 | <4 | 16384 | 2048 | <4 | 128 |
| SEQ ID NO: 3 | <4 | <4 | 2048 | 1024 | 16384 | 4096 |

Within SEQ ID NO: 1 (MC58), therefore, the most important bactericidal epitopes require the presence of both domains B and C (domain BC). This suggests that the protein may include discontinuous epitopes made of sequences from both of these two domains [*cf*. ref. 237].

Moreover, monoclonal antibodies Jar1, Jar3, and Jar4, which are capable of passively protecting rats against meningococcal infection, recognise the BC domain but do not recognise domain B or C alone. Similarly, Jar5 recognises the AB domain, but not domain A or B alone.

Further details of this work can be found in reference 238.

### Monoclonal antibody 502

To select anti-NMB1870 monoclonal antibodies with bactericidal activity, CD1 mice were immunised with family I NMB1870_{MC58}. Polyclonal sera from individual mice were evaluated for antibody binding by ELISA on the purified protein and on whole MC58 cells, and for complement mediated bactericidal activity against MC58. On the basis of these results the spleen of a high responder mouse was selected for the fusion with myeloma cells. Several hybridoma cell lines producing antibodies were isolated and selected by positive ELISA against the purified protein or against MC58 whole bacterial cells. MAb502, an IgG2a isotype monoclonal antibody bactericidal against MC58 strain, was selected for further studies. The antibody recognised the purified protein by ELISA and was positive in FACS analysis on strain MC58.

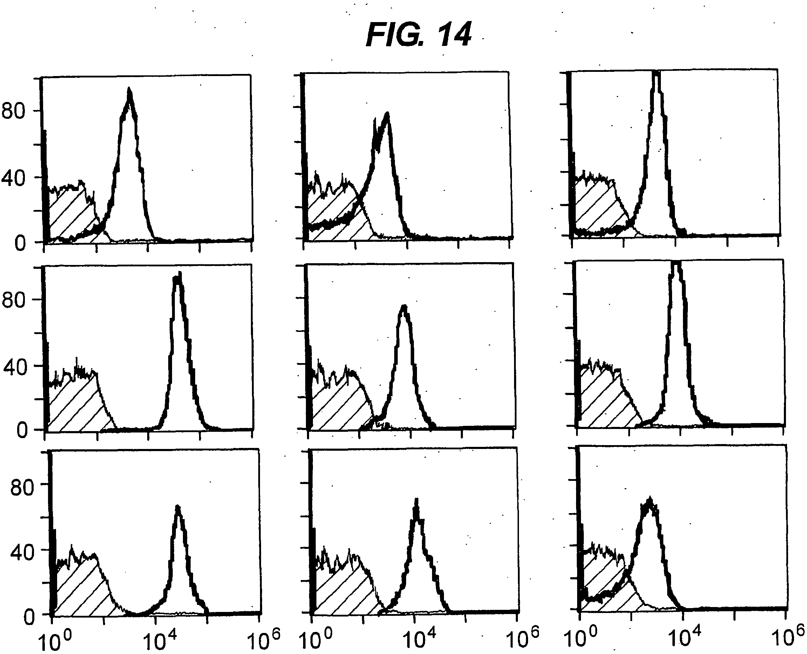

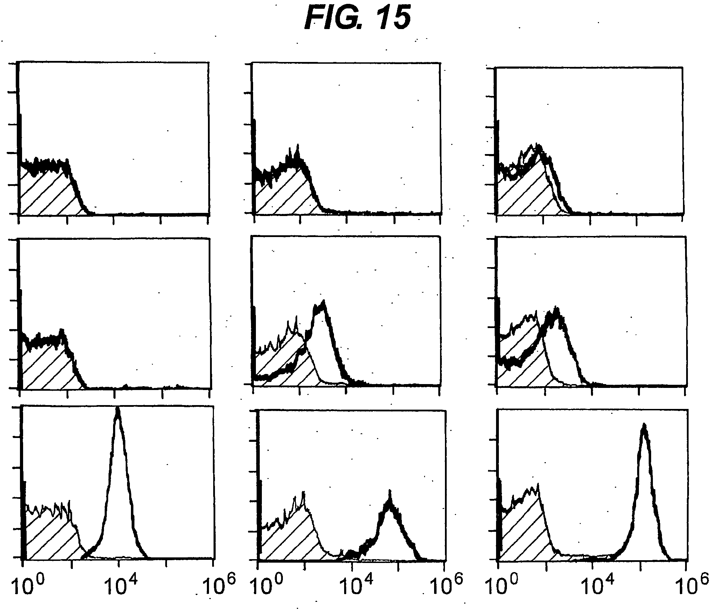

Western blot analysis against NMB1870 from each of the three variants confirmed that mAb502 recognises an epitope present only in family I sequences. In contrast, polyclonal serum recognised all three variants. Monoclonal mAb502 was used in FACS analysis against a number of family I strains. In each case the antibody recognised cell surface antigens (Figure 14, top row). The fluorescence shift was not as great as when using anti-NMB1870 polyclonal (middle row) or using an anti-capsule monoclonal SEAM3 (bottom row), but the binding was specific. In contrast, mAb502 did not recognise a ΔNMB1870 knockout strain (Figure 15, left column) and did not recognise family II or family III strains (middle and right columns). The anti-capsule positive control recognised all of the unrecognised strains (Figure 15, bottom row).

### Comparison of immunogenicity of domains

The A, B and C domains of the MC58ΔG-NMB1870 sequence (family I) were prepared singly and as AB and BC fusions, all with C-terminal His-tags. They were used to immunise mice and sera were tested for bactericidal activity against a strain from each NMB1870 family. For comparison, sera raised in response to the three families' ΔG-NMB1870 sequences were also tested. Proteins were adjuvanted either with an aluminium hydroxide or with FCA. Results were as follows:

| | | **MC58** **B:15:P1.7,16b** | **961-5945** **B:2b:P1.21,16** | **M1239** **B:14:P1.23,14** |
|---|---|---|---|---|
| | **Adjuvant** | ET5 | A4 | lin.3 |
| A | FCA | <4 | <4 | <4 |
| A | AH | 16 | <4 | <4 |
| B | FCA | <4 | <4 | <4 |
| B | AH | <4 | <4 | 256 |
| C | FCA | <4 | <4 | <4 |
| C | AH | 512 | <4 | <4 |
| | | | | |
| AB | FCA | <4 | 256 | <4 |
| AB | AH | <4 | 256 | <4 |
| BC | FCA | 32768 | <4 | <4 |
| BC | AH | 8192 | <4 | <4 |
| | | | | |
| MC58 | FCA | 524288 | 2048 | <4 |
| MC58 | AH | 16384 | <8 | <4 |
| 2996 | FCA | <4 | 16384 | <4 |
| 2996 | AH | <4 | 2048 | 128 |
| M1239 | FCA | <4 | 2048 | 16384 |
| M1239 | AH | <4 | 1024 | 4096 |

Thus the individual domains are not particularly effective immunogens, the AB domain is also not particularly effective, but the BC domain shows good activity.

### 3D model of BC domain

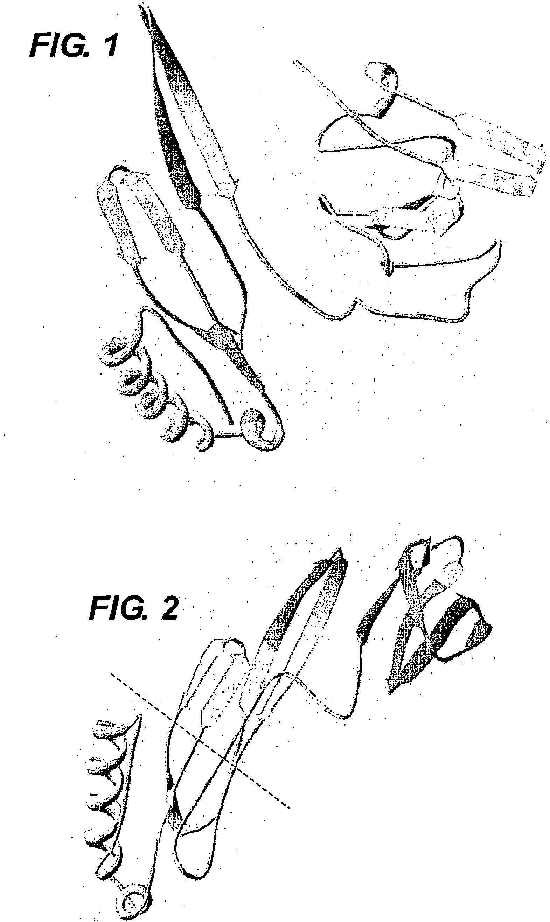

A prediction of super-secondary structure for the BC domain was obtained by submitting the MC58 sequence to the HMMSTR/Rosetta server [240]. The output is shown in Figure 1.

The 3D structure was subjected to a VAST search [241] to find similarity to solved protein structures and to refine loops. The VAST output (Figure 2) was:

| | | |
|---|---|---|
| NMB1870 | | RHAVISGSVLYNQa--EKGSYS1g----iFGGKa-----QEVA |
| 1K32_A | 311 | IAFVSRGQAFIQDvsgTYVI,KVpeplrirYVRRggdtkvAFIH 353 |

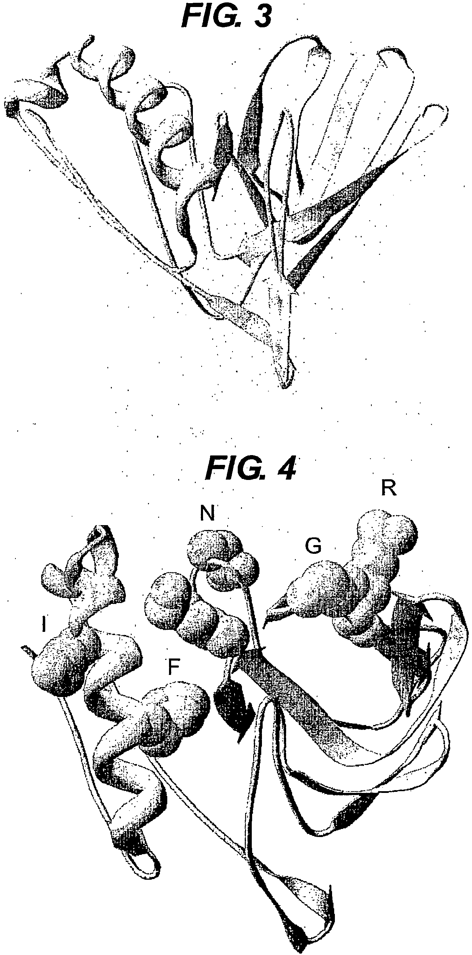

Using the VAST output, the 229-259 fragment (top-right of Figure 2) was further modeled and modified by introducing turns in the backbone along the dashed line of Figure 2. The resulting model was refined by energy minimization, to give the final model of Figure 3.

This model is shown with surface loops highlighted in Figure 6.

### Surface epitope mapping

A multiple sequence alignment of the BC domains of NMB1870 sequences from strains recognised in western blots mAb502 revealed various sequence information. All strains that are bound by the antibody include residue Arg223, but this residue is His in strains that are not recognised in the blots. Even so, not all of the sequences with Erg223 produce bactericidal sera, and so the total bactericidal epitope must be broader than this single residue.

The alignment identified other residues that could be involved in the formation of a specific bactericidal epitope: Phe128 (F), Ile133 (I), Asn197 (N), Gly2217 (G), Lys249 (K), Lys260 (K) and Val262 (V). All these amino acids (grey backgrounds below) are perfectly conserved among ET-5 strains (such as MC58), which are BCA-positive, but differ in the BCA-negative strains:

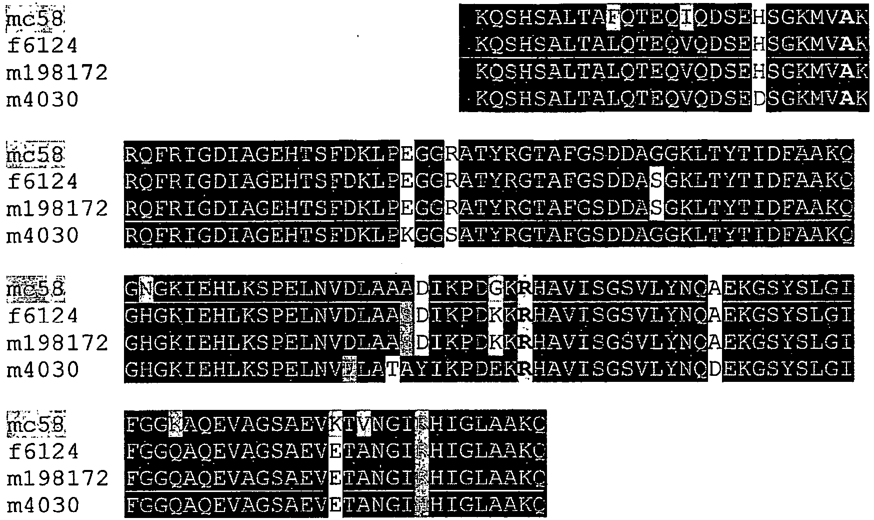

Similar work based on aligning sequences from strains that react with a bactericidal polyclonal serum identified the following residues: Phe128, Ile133, Asn197 and Gly221. Residues Lys249, Lys260 and Va1262, which were identified by the monoclonal antibody, were discarded at this stage, as these three residues were conserved in the NMB1870 sequence of a BCA-negative strain (M2197).

From the 3D model of NMB1870, Phe134 and Ile139 are seen to lie within a predicted alpha-helix, and therefore not well accessible to antibodies. On the other hand, both the Asn197 and Gly221 are contained within surface loops and are well exposed. Both Gly221 and Asn197 are spatially close to Arg223, and could thus be part of the same epitope. Looking at Gly221, BCA-negative strains often have this small and neutral amino acid substituted with a Glu or Lys, both of which are bulky and charged. This substitution could impair proper recognition and binding of antibody to this epitope.

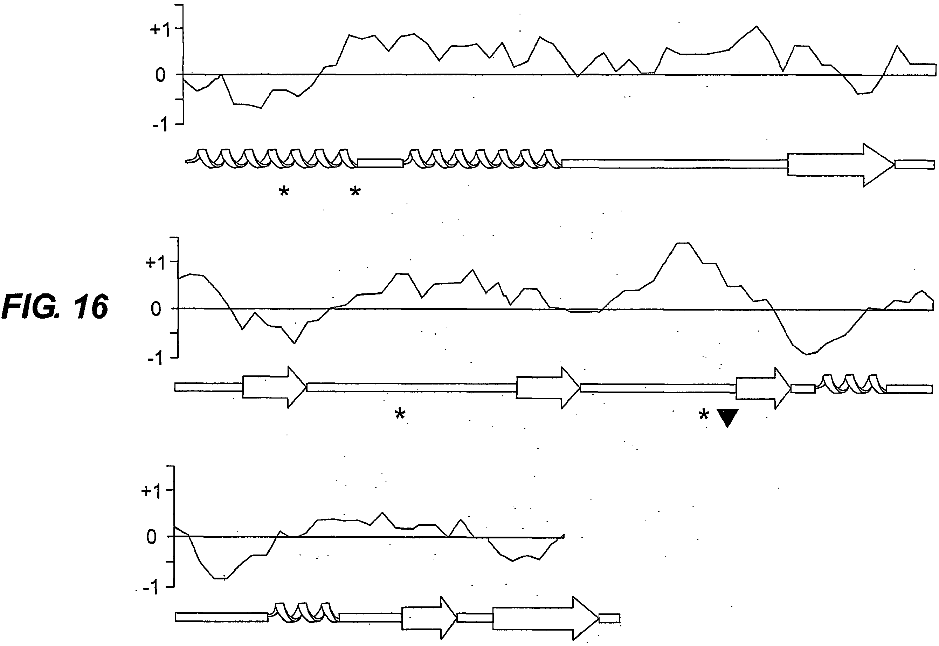

This analysis thus reveals five key amino acids: Phe128, Ile133, Asn197, Gly221 and Arg223. When these residues are mapped to the 3D model (Figure 4), three of them cluster at the protein surface (Figure 5). Alignment with a secondary structure prediction (Figure 16) also shows that Ile133, Asn197, Gly221 and Arg223 are located in hydrophilic regions (i.e. in surface loops).

Other important residues could be the dipeptide AD in position 215-216, which is substituted in most BCA-negative strains by AY, and by S D in some weakly responders.

Amino acids 197, 221 and 223 were mutated as follows:

| **Original amino acid(s)** | Asn-197 | Gly-221 | Arg-223 | Asn-197 & Gly-221 |
|---|---|---|---|---|
| **Substitution(s)** | His | Lys | His | His & Lys |

Mutagenesis used the GeneTailor™ SDM system from Invitrogen. Internal primers containing codon changes were designed according to the instruction manual specifications, and were as follows:

| **Primers** | **Sequences (SEQ ID NO:)** | **Mutation** |
|---|---|---|
| 741(1)-N197H for | GATTTCGCCGCCAAGCAGGGA**cAC**GGCAAAATCGAA(SEQID NO: 66) | AAC → cAC |
| 741(1)-N197H rev | TCCCTGCTTGGCGGCG**AAA**TCTATGGTGTAGGT(SEQ ID NO: 67) | N → H |
| 741(1)-G221K for | GCCGCCGATATCAAGCCGG**ATaaA**AAACGCCATGCC(SEQ ID NO: 68) | GGA → aaA |
| 741(1)-G221K rev | ATCCGGCTTGATATCGGCGGCGGCCAGGTCGAC(SEQ ID NO: 69) | G → K |
| 741(1)-R223H for | GATATCAAGCCGGATGG**AAAACaC**CATGCCGTCATCAGC (SEQ ID NO: 70) | CGC → CaC |
| 741(1)-R223H rev | TTTCCATCCGGCTTGATATCGGCGGCGGCCAGGTC (SEQ ID NO: 71) | R → H |

To generate each mutant, 100ng of the pET-ΔG741₍₁₎-His plasmid DNA were used as template in a methylation reaction, then 12.5ng of methylated plasmid were employed as substrate in a mutagenesis reaction, using the following primer pairs:
741(1)-N197H for / 741(1)-N197H rev
741(1)-G221K for / 741(1)-G221K rev
741(1)-R223H for / 741(1)-R223H rev

PCR was performed according to the GeneTailor™ Site-Directed Mutagenesis System instruction manual. After the reaction, 10µl of the product were analysed on a 1% agarose gel, then 2µl from mutagenesis reaction mixture were transformed into DH5a™-T1^{R} *E.coli* strain according to manual specifications. Positive colonies were analysed by plasmid isolation (QIAprep Spin Miniprep Kit, QIAGEN™) and sequencing. To generate the double-mutant ΔG741₍₁₎-His-N197H-G221K, the DNA of the positive mutant ΔG741(1)-His-G221K was used as substrate for the next one with the corresponding pair of primers 741₍₁₎-N197H for/741₍₁₎-N197H rev.

For expression of the recombinant protein as C terminal-His-tag fusion, 1.5µl of each construct was used to transform *E.coli* BL21-DE₃ strain. Single recombinant colonies were inoculated into 4ml LB+Amp (100µg/ml), incubated at 37°C overnight, then diluted 1:30 in 20ml of LB+Amp (100µg/ml) in 125ml flasks, to give an OD₆₀₀ₙₘ between 0.1 and 0.2. The flasks were incubated at 37°C in a gyratory water bath shaker until OD₆₀₀ₙₘ indicated exponential growth suitable for induction of expression (0.4-0.8 OD). Protein expression was induced by addition of 1.0mM IPTG. After 3 hours incubation at 37°C the OD₆₀₀ₙₘ was measured and expression examined. 1.0ml of each sample was centrifuged in a microfuge, the pellet resuspended in PBS and analysed by SDS-PAGE and Coomassie Blue staining. All the mutants were expressed as well as wild type and purified as soluble forms at 37°C.

### Loop substitution

Based on the 3D model and on the epitope mapping work, surface loops from family II and family III of NMB1870 were transferred into a family I framework.

For Loop1, the amino acid sequence is 100% conserved among all family II and family III strains. In all the three families, Loop1 is flanked by two alpha helices (not conserved in sequence) that likely contribute to the correct exposure/folding of the loop. The Gly140 residue is not found in the family I M6190 sequence, and while monoclonals Jar3 and Jar5 bind to family I sequences they fail to bind to M6190, further confirming the epitopic importance of this loop. The family II sequence was chosen for insertion into the family I framework.

Loop2 corresponds to a bactericidal epitope. The only difference between families II and III at this position is an Asp/Gly substitution. Analysis of bactericidal responses among family II strains suggests a critical role for the Asp residue, so the family II sequence was chosen.

Loop3 is highly variable within families II and III, but is conserved in family I. In family II, a majority of strains has sequence PNG, but in family III the majority have AGG. Although this loop seems dispensable for protection (PNG strains are able to protect against family III AGG strains), to cover both possibilities the family I AG sequence was substituted with PN.

Asn197 in Loop4 was previously identified as an important residue to discriminate between BCA-positive and BCA-negative strains of family I, and is always conserved in ET-5 strains. This residue is substituted by His in all family II strains and in a subgroup of family III strains. In addition, His is also present in some family I strains. His was thus used in this loop, to cover families II and III, plus any family I sequences not covered by the MC58 sequence.

Loop5, Loop6 and Loop7 are the same in families II and III, but different from family I. For these three loops the family II/III sequences were used. The second residue in the loop is Gly rather than Val because family II strains that are susceptible to serum raised against the strain 2996 protein have this residue at that position.

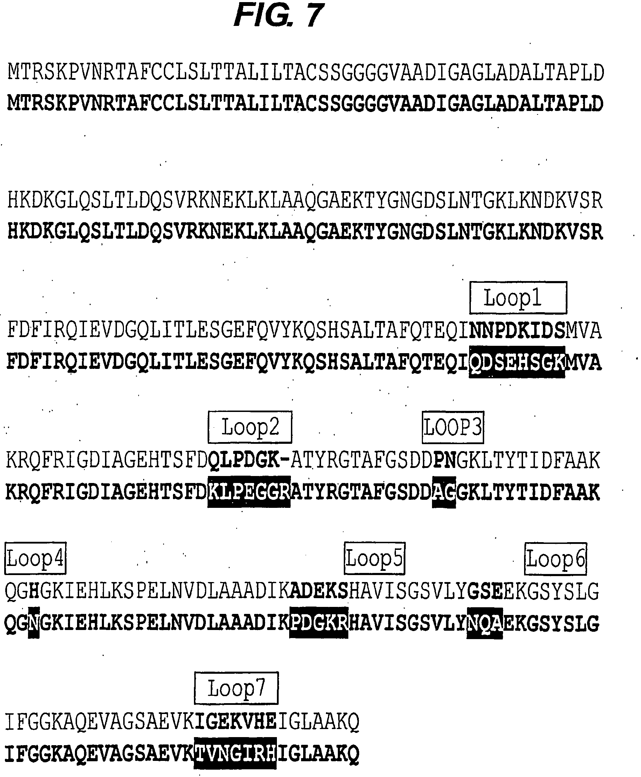

The substitutions are shown in Figure 7, to change SEQ ID NO: 1 into SEQ ID NO: 61. Loops 1, 2 and 4 received family II sequences; loop 3 received the family III sequence; and loops 5, 6 and 7 received a sequence common to both families II and III. With 28 substitutions out of 274 (SEQ ID NO: 1) and 273 (SEQ ID NO: 61) amino acids in total, the overall identity after surface loop exchange remains at >90%.

The loops were substituted in series. Seven proteins were made in this series, with each loop of the ΔGNMB1870_{MC58} sequence being substituted in order. The seven substituted proteins were referred to as LP₁, LP₂, ..., LP₇. The LP₇ mutant is SEQ ID NO: 61.

The GeneTailor™ SDM system was used for this work (see above), with the following primers:

| **Primers** | **Sequences** | **SEQ ID NO** |
|---|---|---|
| 741(1)-LP1 for | GCCTTTCAGACCGAGCAAATA**aAcaAccCGGAcaAaatCGacAgcA**TGGTTGCGAAACGC | 72 |
| 741(1)-LP1 rev | TATTTGCTCGGTCTGAAAGGCGGTTAAGGCGGA | 73 |
| 741(1)-LP2 for | GGCGAACATACATCTTTTGAC**cAGCTTCCCGAcGGCaaa**AGGGCGACATATCGC | 74 |
| 741(1)-LP2 rev | GTCAAAAGATGTATGTTCGCCCGCTATGTCGCC | 75 |
| 741(1)-LP3 for | ACGGCGTTCGGTTCAGACGAT**cCgaaC**GGAAAACTGACCTAC | 76 |
| 741(1)-LP3 rev | ATCGTCTGAACCGAACGCCGTCCCGCGATATGTCGC | 77 |
| 741(1)-LP4 for | GATTTCGCCGCCAAGCAGGGA**cAC**GGCAAAATCGAA | 78 |
| 741(1)-LP4 rev | TCCCTGCTTGGCGGCGAAATCTATGGTGTAGGT | 79 |
| 741(1)-LP5 for | CTGGCCGCCGCCGATATCAAG**gCcGATGaAAAAaGC**CATGCCGTCATC | 80 |
| 741(1)-LP5 rev | CTTGATATCGGCGGCGGCCAGGTCGACATTGAG | 81 |
| 741(1)-LP6 for | ATCAGCGGTTCCGTCCTTTAC**ggCagcGaa**GAGAAAGGCAGT | 82 |
| 741(1)-LP6 rev | GTAAAGGACGGAACCGCTGATGACGGCATGGCG | 14 |
| 741(1)-LP7 for | GCCGGCAGCGCGGAAGTGAAA**AtCGgcgAaaaggTACaCgAaA**TCGGCCTTGCCGCC | 15 |
| 741(1)-LP7 rev | TTTCACTTCCGCGCTGCCGGCAACTTCCTGGGCTTT | 16 |

All proteins were expressed as well as the wild-type protein and could be purified as soluble products after growth at 37°C. The proteins LP₁, LP₂, ..., LP₇ were used to immunise mice, and the resulting sera were tested against various different strains of serogroup B meningococcus, including at least three from each NMB1870 family. The starting protein (LP0) was also tested. The proteins were adjuvanted either with an aluminium hydroxide (AH) adjuvant or with FCA (F). In the following table, each row (except for the bottom row) shows the SBA for a single serum, tested against nine exemplary strains. The bottom row shows the activity of a serum obtained using the wild-type antigen from the homologous NMB1870 (i.e.. testing anti-NMB1870_{MC58} serum against MC58, *etc*.):

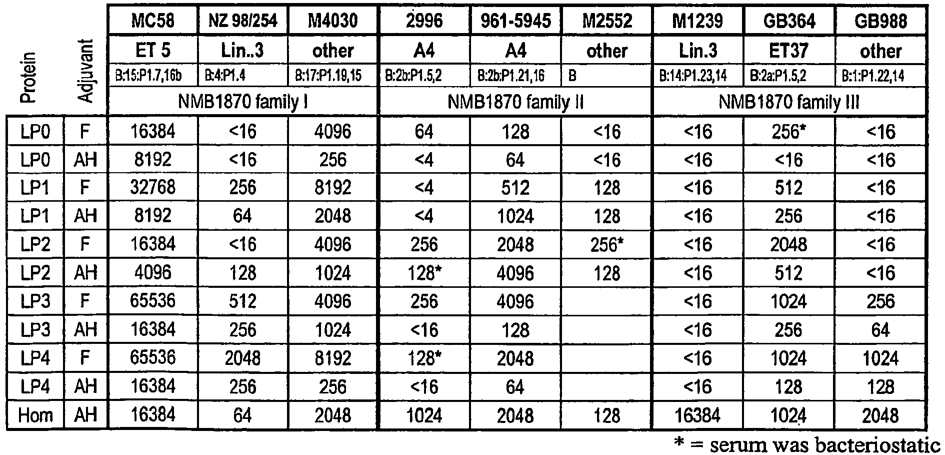

Progressing from LP0 (no substitutions; NMB1870; family I) to LP7 through LP1, LP2, *etc.,* sera raised against the protein become more active against strains whose NMB1870 is in family II or family III. As family I sequences are substituted with family II/III sequences then, contrary to a priori expectations, bactericidal responses against family I strains show an upward trend.

Thus SEQ ID NO: 61 contains surface epitopes from families II and III, to replace family I epitopes. The chimeric polypeptide can be used to raise antibodies against NMB1870 from families II and III, and can be combined with a normal family I sequence to give multi-family antigenicity. The combination may be as a mixture of two separate polypeptides, or may be in the form of a hybrid [7] *e.g*. NH₂-X₁-SEQ:61-X₂-SEQ:1-X₃-COOH, NH₂-X₁-SEQ:1-X₂-SEQ:61-X₃-COOH, *etc.*

### New NMB1870 sequences

Extensive sequence information for NMB1870 is available [e.g. refs 3, 5, 6 and 7]. Further new NMB1870 sequences have been found.

The sequence for strain 4243 is given as SEQ ID NO: 62, starting at the N-terminal cysteine of the mature protein. The cleaved leader peptide is the same as a normal family I sequence.

A fourth family of NMB1870 has been seen in strain M.01.0240320 ('gb320'; SEQ ID NO: 63) and in strain S10026 (SEQ ID NO: 64). The sequence from m3813 (SEQ ID NO: 21 of ref. 7; SEQ ID NO: 65 herein) can also be classified into family IV.

### BRIEF DESCRIPTION OF THE SEQUENCE LISTING

| **SEQ ID NO:** | **Description** |
|---|---|
| 1 | NMB 1870 from strain MC58 - family I |
| 2 | NMB 1870 from strains 961-5945 & 2996 - family II |
| 3 | NMB 1870 from strain M1239 - family III |
| 4-6 | Domains A to C from SEQ ID NO: 1 |
| 7-9 | Domains A to C from SEQ ID NO: 2 |
| 10-12 | Domains A to C from SEQ ID NO: 3 |
| 13 | mature domain A from SEQ ID NO: 4 |
| 14-16 | SDM primers |
| 17-20 | Linkers & expression sequences |
| 21-22 | Common epitopes |
| 23 | AB |
| 24 | BC |
| 25-42 | Primers |
| 43 | B_{M1239}C_{MC58} |
| 44 | BC₂₉₉₆BC_{M1239} |
| 45 | Linker |
| 46-51 | Primers |
| 52 | BC_{MC58}-BC_{M1239}-BC₂₉₉₆ |
| 53 | NMB1870_{MC58}-NMB1870_{M1239}-NMB1870₂₉₉₆ |
| 54 | Sequence for expression |
| 55-60 | Primers |
| 61 | Surface loop substitution |
| 62-65 | NMB1870 sequences |
| 66-82 | SDM Primers |

### REFERENCES

[1] Jodar et al. (2002) Lancet 359(9316):1499-1508.
[2] WO99/57280.
[3] Masignani et al. (2003) J Exp Med 197:789-799.
[4] Pizza et al. (2000) Science 287:1816-1820.
[5] WO03/063766.
[6] Fletcher et al. (2004) Infect Immun 72:2088-2100.
[7] WO2004/048404.
[8] Achtman (1995) Global epidemiology of meningococcal disease. Pages 159-175 of Meningococcal disease (ed. Cartwight). ISBN: 0-471-95259-1.
[9] Caugant (1998) APMIS 106:505-525.
[10] Maiden et al. (1998) Proc. Natl. Acad. Sci. USA 95:3140-3145.
[11] Needleman & Wunsch (1970) J. Mol. Biol. 48, 443-453.
[12] Rice et al. (2000) Trends Genet 16:276-277.
[13] WO01/30390.
[14] Gennaro (2000) Remington: The Science and Practice of Pharmacy. 20th edition, ISBN: 0683306472.
[15] WO03/009869.
[16] Vaccine Design... (1995) eds. Powell & Newman. ISBN: 030644867X. Plenum.
[17] WO00/23105.
[18] WO90/14837.
[19] US patent 5,057,540.
[20] WO96/33739.
[21] EP-A-0109942.
[22] WO96/11711.
[23] WO00/07621.
[24] Barr et al. (1998) Advanced Drug Delivery Reviews 32:247-271.
[25] Sjolanderet et al. (1998) Advanced Drug Delivery Reviews 32:321-338.
[26] Niikura et al. (2002) Virology 293:273-280.
[27] Lenz et al. (2001) J Immunol 166:5346-5355.
[28] Pinto et al. (2003) J Infect Dis 188:327-338.
[29] Gerber et al. (2001) Virol 75:4752-4760.
[30] WO03/024480
[31] WO03/024481
[32] Gluck et al. (2002) Vaccine 20:B10-B16.
[33] EP-A-0689454.
[34] Johnson et al. (1999) Bioorg Med Chem Lett 9:2273-2278.
[35] Evans et al. (2003) Expert Rev Vaccines 2:219-229.
[36] Meraldi et al. (2003) Vaccine 21:2485-2491.
[37] Pajak et al. (2003) Vaccine 21:836-842.
[38] Kandimalla et al. (2003) Nucleic Acids Research 31:2393-2400.
[39] WO02/26757.
[40] WO99/62923.
[41] Krieg (2003) Nature Medicine 9:831-835.
[42] McCluskie et al. (2002) FEMS Immunology and Medical Microbiology 32:179-185.
[43] WO98/40100.
[44] US patent 6,207,646.
[45] US patent 6,239,116.
[46] US patent 6,429,199.
[47] Kandimalla et al. (2003) Biochemical Society Transactions 31 (part 3):654-658.
[48] Blackwell et al. (2003) J Immuol 170:4061-4068.
[49] Krieg (2002) Trends Immunol 23:64-65.
[50] WO01/95935.
[51] Kandimalla et al. (2003) BBRC 306:948-953.
[52] Bhagat et al. (2003) BBRC 300:853-861.
[53] WO03/035836.
[54] WO95/17211.
[55] WO98/42375.
[56] Beignon et al. (2002) Infect Immun 70:3012-3019.
[57] Pizza et al. (2001) Vaccine 19:2534-2541.
[58] Pizza et al. (2000) Int J Med Microbiol 290:455-461.
[59] Scharton-Kersten et al. (2000) Infect Immun 68:5306-5313.
[60] Ryan et al. (1999) Infect Immun 67:6270-6280.
[61] Partidos et al. (1999) Immunol Lett 67:209-216.
[62] Peppoloni et al. (2003) Expert Rev Vaccines 2:285-293.
[63] Pine et al. (2002) J Control Release 85:263-270.
[64] Domenighini et al. (1995) Mol Microbiol 15:1165-1167.
[65] WO99/40936.
[66] WO99/44636.
[67] Singh et al] (2001) J Cont Release 70:267-276.
[68] WO99/27960.
[69] US patent 6,090,406
[70] US patent 5,916,588
[71] EP-A-0626169.
[72] WO99/52549.
[73] WO01/21207.
[74] WO01/21152.
[75] Andrianov et al. (1998) Biomaterials 19:109-115.
[76] Payne et al. (1998) Adv Drug Delivery Review 31:185-196.
[77] Stanley (2002) Clin Exp Dermatol 27:571-577.
[78] Jones (2003) Curr Opin Investig Drugs 4:214-218.
[79] WO99/11241.
[80] WO94/00153.
[81] WO98/57659.
[82] European patent applications 0835318, 0735898 and 0761231.
[83] WO99/24578.
[84] WO99/36544.
[85] Costantino et al. (1992) Vaccine 10:691-698.
[86] Costantino et al. (1999) Vaccine 17:1251-1263.
[87] WO03/007985.
[88] Watson (2000) Pediatr Infect Dis J 19:331-332.
[89] Rubin (2000) Pediatr Clin North Am 47:269-285, v.
[90] Jedrzejas (2001) Microbiol Mol Biol Rev 65:187-207.
[91] Bell (2000) Pediatr Infect Dis J 19:1187-1188.
[92] Iwarson (1995) APMIS 103:321-326.
[93] Gerlich et al. (1990) Vaccine 8 Suppl:S63-68 & 79-80.
*[94]* Vaccines (1988) eds. Plotkin & Mortimer. ISBN 0-7216-1946-0.
[95] Del Guidice et al. (1998) Molecular Aspects of Medicine 19:1-70.
[96] Gustafsson et at. (1996) N. Engl. J. Med. 334:349-355.
[97] Rappuoli et al. (1991) TIBTECH 9:232-238.
[98] Sutter et al. (2000) Pediatr Clin North Am 47:287-308.
[99] Zimmerman & Spann (1999) Am Fam Physician 59:113-118, 125-126.
[100] McMichael (2000) Vaccine 19 Suppl 1:S101-107.
[101] Schuchat (1999) Lancet 353(9146):51-6.
[102] WO02/34771.
[103] Dale (1999) Infect Dis Clin North Am 13:227-43, viii.
[104] Ferretti et al. (2001) PNAS USA 98: 4658-4663.
[105] Kuroda et al. (2001) Lancet 357(9264):1225-1240; see also pages 1218-1219.
[106] EP-A-0372501
[107] EP-A-0378881
[108] EP-A-0427347
[109] WO93/17712
[110] WO94/03208
[111] WO98/58668
[112] EP-A-0471177
[113] WO00/56360
[114] WO91/01146
[115] WO00/61761
[116] WO01/723 37
[117] Research Disclosure, 453077 (Jan 2002)
[118] Jones (2001) Curr Opin Investig Drugs 2:47-49.
[119] Ravenscroft et al. (1999) Vaccine 17:2802-2816.
[120] WO03/080678.
[121] Nilsson & Svensson (1979) Carbohydrate Research 69: 292-296)
[122] Frash (1990) p.123-145 of Advances in Biotechnological Processes vol. 13 (eds. Mizrahi & Van Wezel)
[123] Inzana (1987) Infect. Immun. 55:1573-1579.
[124] Kandil et al. (1997) Glycoconj J 14:13-17.
[125] Berkin et al. (2002) Chemistry 8:4424-4433.
[126] Lindberg (1999) Vaccine 17 Suppl 2:S28-36.
[127] Buttery & Moxon (2000) J R Coll Physicians Lond 34:163-168.
[128] Ahmad & Chapnick (1999) Infect Dis Clin North Am 13:113-33, vii.
[129] Goldblatt (1998) J. Med. Microbiol. 47:563-567.
[130] European patent 0477508.
[131] US patent 5,306,492.
[132] WO98/42721.
[133] Dick et al. in Conjugate Vaccines (eds. Cruse et al.) Karger, Basel, 1989, 10:48-114.
[134] Hermanson Bioconjugate Techniques, Academic Press, San Diego (1996) ISBN: 0123423368.
[135] Kanra et al. (1999) The Turkish Journal of Paediatrics 42:421-427.
[136] Ravenscroft et al. (2000) Dev Biol (Basel) 103: 35-47.
[137] WO97/00697.
[138] WO02/00249.
[139] Zielen et al. (2000) Infect. Immun. 68:1435-1440.
[140] Darkes & Plosker (2002) Paediatr Drugs 4:609-630.
[141] Tettelin et al. (2001) Science 293:498-506.
[142] Hoskins et al (2001) J Bacteriol 183:5709-5717.
[143] Rappuoli (2000) Curr Opin Microbiol 3:445-450
[144] Rappuoli (2001) Vaccine 19:2688-2691.
[145] Masignani et al. (2002) Expert Opin Biol Ther 2:895-905.
[146] Mora et al. (2003) Drug Discov Today 8:459-464.
[147] Wizemann et al. (2001) Infect Immun 69:1593-1598.
[148] Rigden et al. (2003) Crit Rev Biochem Mol Biol 38:143-168.
[149] WO02/22167.
[150] Ramsay et al. (2001) Lancet 357(9251):195-196.
[151] Anderson (1983) Infect Immun 39(1):233-238.
[152] Anderson et al. (1985) J Clin Invest 76(1):52-59.
[153] Falugi et al. (2001) Eur J Immunol 31:3816-3824.
[154] EP-A-0594610.
[155] WO02/091998.
[156] WO99/42130
[157] WO96/40242
[158] Lees et al. (1996) Vaccine 14:190-198.
[159] WO95/08348.
[160] US patent 4,882,317
[161] US patent 4,695,624
[162] Porro et al. (1985) Mol Immunol 22:907-919.s
[163] EP-A-0208375
[164] WO00/10599
[165] Gever et al. Med. Microbiol. Immunol, 165 : 171-288 (1979).
[166] US patent 4,457,685.
[167] US patents 4,673,574; 4,761,283; 4,808,700.
[168] US patent 4,459,286.
[169] US patent 4,965,338
[170] US patent 4,663,160.
[171] US patent 4,761,283
[172] US patent 4,356,170
[173] Lei et al. (2000) Dev Biol (Basel) 103:259-264.
[174] WO00/38711; US patent 6,146,902.
[115] WO02/09643.
[176] Katial et al. (2002) Infect Immun 70:702-707.
[177] WO01/52885.
[178] European patent 0301992.
[179] Bjune et al. (1991) Lancet 338(8775):1093-1096.
[180] Fukasawa et al. (1999) Vaccine 17:2951-2958.
[181] WO02/09746.
[182] Rosenqvist et al. (1998) Dev. Biol. Stand. 92:323-333.
[183] WO01/09350.
[184] European patent 0449958.
[185] EP-A-0996712.
[186] EP-A-0680512.
[187] WO02/062378.
[188] WO99/59625.
[189] US patent 6,180,111.
[190] WO01/34642.
[191] WO03/051379.
[192] US patent 6,558,677.
[193] WO2004/019977.
[194] WO02/062380.
[195] WO00/25811.
[196] Peeters et al. (1996) Vaccine 14:1008-1015.
[197] Vermont et al. (2003) Infect Immun 71:1650-1655.
[198] Kabat et al., 1987, in Sequences of Proteins of Immunological Interest, US Department of Health and Human Services, NIH, USA
[199] Breedveld (2000) Lancet 355(9205):735-740.
[200] Gorman & Clark (1990) Semin, Immunol. 2:457-466
[201] Jones et al. (1986) Nature 321:522-525
[202] Morrison et al. (1984) Proc. Natl. Acad. Sci, USA., 81:6851-6855
[203] Morrison & Oi, (1988) Adv. Immunol., 44:65-92.
[204] Verhoeyer et al. (1988) Science 239:1534-36.
[205] Padlan (1991) Molec. Immun. 28:489-98.
[206] Padlan (1994) Molec. Immunol. 31:169-217.
[207] Kettleborough et al. (1991) Protein Eng. 4:773-83.
[208] WO98/24893
[209] WO91/10741
[210] WO96/30498
[211] WO94/02602
[212] US Patent 5,939,598.
[213] Conrath et al. (2003) Dev Comp Immunol 27:87-103.
[214] Muyldermans (2001) J Biotechnol 74:277-302.
[215] Huston et al. (1988) Proc. Nat. Acad. Sci. USA 85 :5879-5883.
[216] US Patent 5,091,513
[217] US Patent 5,132,405
[218] US Patent 4,946,778
[219] Pack et al., (1992) Biochem 31:1579-1584
[220] Cumber et al. (1992) J. Immunology 149B:120-126
[221] Radrizzani M et al., (1999) Medicina (B Aires) 59(6):753-8.
[222] Radrizzani M et al. (2000) Medicina (B Aires) 60 Suppl 2:55-60.
[223] US Patent 4,011,308
[224] US Patent 4,722,890
[225] US Patent 4,016,043
[226] US Patent 3,876,504
[227] US Patent 3,770,380
[228] US Patent 4,372,745
[229] Kohler & Milstein (1975) Nature 256:495-497
[230] Inbar et al. (1972) Proc. Nat. Acad. Sci USA 69:2659-2662
[231] Hochman et al. (1976) Biochem 15:2706-2710
[232] Ehrlich et al. (1980) Biochem 19:4091-4096
[233] Siegel, Transfus. Clin. Biol. (2002) 9(1): 15-22;
[234] Sidhu, Curr. Opin. Biotechnol. (2000) 11(6):610-616;
[235] Sharon, et al., Comb. Chem. High Throughput Screen (2000) 3(3): 185-196;
[236] Schmitz et al., Placenta, (2000) 21 Supp1A: S106-12
[237] Bartoloni et al. (1988) Bio/technology 6:709-712.
[238] Giuliani et al. (2005) Infect Immun 73:1151-60.
[239] WO2004/032958.
[240] *http:*//*www.bioinfo.rpi.edu*/*∼bystrc*/*hmmstr*/*sever.php*
[241] *http:*//*www.ncbi.nlm.nih.gov*/*Structure*/*VAST*/*vast.shtml*

### SEQUENCE LISTING

***SEQ ID NO: 1 - strain MC58 - family I*** 
MNRTAFCCLSLTTALILTACSSGGGGVAADIGAGLADALTAPLDHKDKGLQSLTLDQSVRKNEKLKLAAQGAEKTYGNGDSLNTGKLKNDKVSRFD
FIRQIEVDGQLITLESGEFQVYKQSHSALTAFQTEQIQDSEHSGKMVAKRQFRIGDIAGEHTSFDKLPEGGRATYRGTAFGSDDAGGKLTYTIDFA
AKQGNGKIEHLKSPELNVDLAAADIKPDGKRHAVISGSVLYNQAEKGSYSLGIFGGKAQEVAGSAEVKTVNGIRHIGLAAKQ
***SEQ ID NO: 2 - strains 961-5945 & 2996 - family II*** 
MNRTAFCCLSLTAALILTACSSGGGGVAADIGAGLADALTAPLDHKDKSLQSLTLDQSVRKNEKLKLAAQGAEKTYGNGDSLNTGKLKNDKVSRFD
FIRQIEVDGQLITLESGEFQIYKQDHSAVVALQIEKINNPDKIDSLINQRSFLVSGLGGEHTAFNQLPDGKAEYHGKAFSSDDAGGKLTYTIDFAA
KQGHGKIEHLKTPEQNVELAAAELKADEKSHAVILGDTRYGSEEKGTYHLALFGDRAQEIAGSATVKIGEKVHEIGIAGKQ
***SEQ ID NO: 3 - strain M1239 - family III*** 
MNRTAFCCLSLTTALILTACSSGGGGSGGGVAADIGTGLADALTAPLDHKDKGLKSLTLEDSIPQNGTLTLSAQGAEKTFKAGDKDNSLNTGKLK
NDKISRFDFVQKIEVDGQTITLASGEFQIYKQNHSAVVALQIEKINNPDKTDSLINQRSFLVSGLGGEHTAFNQLPGGKAEYHGKAFSSDDPNGRL
HYSIDFTKKQGYGRIEHLKTLEQNVELAAAELKADEKSHAVILGDTRYGSEEKGTYHLALFGDRAQEIAGSATVKIGEKVHEIGIAGKQ
***SEQ ID NO: 4 - domain A from SEQ ID NO: 1*** 
MNRTAFCCLSLTTALILTACSSGGGGVAADIGAGLADALTAPLDHKDKGLQSLTLDQSVRKNEKLKLAAQGAEKTYGNGDSLNTGKLKNDKVSRFD
FIRQIEVDGQLITLESGEFQVYK
***SEQ ID NO: 5 - domain B from SEQ ID NO: 1***
   QSHSALTAFQTEQIQDSEHSGKMVAKRQFRIGDIAGEHTSFDKLPEGGRATYRGTAFGSDDAGG
***SEQ ID NO: 6 - domain C from SEQ ID NO: 1***
   KLTYTIDFAAKQGNGKIEHLKSPELNVDLAAADIKPDGKRHAVISGSVLYNQAEKGSYSLGIFGGKAQEVAGSAEVKTVNGIRHIGLAAKQ
***SEQ ID NO:* 7 - *domain A from SEQ ID NO: 2*** 
MNRTAFCCLSLTAALILTACSSGGGGVAADIGAGLADALTAPLDHKDKSLQSLTLDQSVRKNEKLKLAAQGAEKTYGNGDSLNTGKLKNDKVSRFD
FIRQIEVDGQLITLESGEFQIYK
***SEQ ID NO: 8 - domain B from SEQ ID NO: 2***
   QDHSAVVALQIEKINNPDKIDSLINQRSFLVSGLGGEHTAFNQLPDGKAEYHGKAFSSDDAGG
***SEQ ID NO: 9 - domain C from SEQ ID NO: 2***
   KLTYTIDFAAKQGHGKIEHLKTPEQNVELAAAELKADEKSHAVILGDTRYGSEEKGTYHLALFGDRAQEIAGSATVKIGEKVHEIGIAGKQ
***SEQ ID NO: 10 - domain A from SEQ ID NO: 3*** 
MNRTAFCCLSLTTALILTACSSGGGGSGGGGVAADIGTGLADALTAPLDHKDKGLKSLTLEDSIPQNGTLTLSAQGAEKTFKAGDKDNSLNTGKLK
NDKISRFDFVQKIEVDGQTITLASGEFQIYK
***SEQ ID NO: 11 - domain B from SEQ ID NO: 3***
   QNHSAVVALQIEKINNPDKTDSLINQRSFLVSGLGGEHTAFNQLPGGKAEYHGKAFSSDDPNG
***SEQ ID NO: 12 - domain C from SEQ ID NO: 3***
   RLHYSIDFTKKQGYGRIEHLKTLEQNVELAAAELKADEKSHAVILGDTRYGSEEKGTYHLALFGDRAQEIAGSATVKIGEKVHEIGIAGKQ
***SEQ ID NO: 13 - mature domain A from SEQ ID NO: 4*** 
CSSGGGGVAADIGAGLADALTAPLDHKDKGLQSLTLDQSVRKNEKLKLAAQGAEKTYGNGDSLNTGKLKNDKVSRFDFIRQIEVDGQLITLESGEF
QVYK
***SEQ ID NO: 14 - SDM primer***
   GTAAAGGACGGAACCGCTGATGACGGCATGGCG
***SEQ ID NO: 15 - SDMprimer***
   GCCGGCAGCGCGGAAGTGAAAATCGGCGAAAAGGTACACGAAATCGGCCTTGCCGCC
***SEQ ID NO: 16 - SDM primer***
   TTTCACTTCCGCGCTGCCGGCAACTTCCTGGGCTTT
***SEQ ID NO: 17***
   GSGGGG
***SEQ ID NO: 18***
   GGGG
***SEQ ID NO: 19 - Sequence for expression***
   GSGPDSDRLQQRR
***SEQ ID NO: 20 - Sequence for expression***
   GPDSDRLQQRR
***SEQ ID NO: 21 - common epitope***
   DKGLQSLTLDQSVR
***SEQ ID NO: 22 - common epitope***
   FDFIRQIEVDGQLI
***SEQ ID NO: 23 -AB*** 
MNRTAFCCLSLTTALILTACSSGGGGVAADIGAGLADALTAPLDHKDKGLQSLTLDQSVRKNEKLKLAAQGAEKTYGNGDSLNTGKLKNDKVSRFD
FIRQIEVDGQLITLESGEFQVYKQSHSALTAFQTEQIQDSEHSGKMVAKRQFRIGDIAGEHTSFDKLPEGGRATYRGTAFGSDDAGG
***SEQ ID NO: 24 - BC*** 
QSHSALTAFQTEQIQDSEHSGKMVAKRQFRIGDIAGEHTSFDKLPEGGRATYRGTAFGSDDAGGKLTYTIDFAAKQGNGKIEHLKSPELNVDLAAA
DIKPDGKRHAVISGSVLYNQAEKGSYSLGIFGGKAQEVAGSAEVKTVNGIRHIGLAAKQ
***SEQ ID NO: 25 - oligonucleotide primer***
   CGCGGATCCCATATGGTCGCCGCCGACATCG
***SEQ ID NO: 26 - oligonucleotide primer***
   CCCGCTCGAGTTGTTTGTATACTTGGAACTCTCCAC
***SEQ ID NO: 27 - oligonucleotide primer***
   CGCGGATCCCATATGCAAAGCCATTCCGCCTTAA
***SEQ ID NO: 28 - oligonucleotide primer***
   CCCGCTCGAGTCCGCCGGCATCGTCTG
***SEQ ID NO: 29 - oligonucleotide primer***
   CGCGGATCCCATATGGGAAAACTGACCTACACCA
***SEQ ID NO: 30 - oligonucleotide primer***
   CCCGCTCGAGTTGCTTGGCGGCAAGGC
***SEQ ID NO: 31 - oligonucleotide primer***
   CGCGGATCCCATATGGTCGCCGCCGACATCG
***SEQ ID NO: 32 - oligonucleotide primer***
   CCCGCTCGAGTCCGCCGGCATCGTCTG
***SEQ ID NO: 33 - oligonucleotide primer***
   CGCGGATCCCATATGCAAAGCCATTCCGCCTTAA
***SEQ ID NO: 34 - oligonucleotide primer***
   CCCGCTCGAGTTGCTTGGCGGCAAGGC
***SEQ ID NO: 35 - oligonucleotide primer***
   CGCGGATCCCATATGCAGAACCACTCCGCCGT
***SEQ ID NO: 36 - oligonucleotide primer***
   GCCCAAGCTTGCCATTCGGGTCGTCGG
***SEQ ID NO: 37- oligonucleotide primer***
   GCCCAAGCTTAAACTGACCTACACCATAGA
***SEQ ID NO: 38 - oligonucleotide primer***
   CCCGCTCGAGTTGCTTGGCGGCAAGGC
***SEQ ID NO: 39 - oligonucleotide primer***
   CGCGGATCCCATATGCAGGACCACTCCGCCG
***SEQ ID NO: 40 - oligonucleotide primer***
   CGCGGATCCCTGTTTGCCGGCGATGCC
***SEQ ID NO: 41- oligonucleotide primer***
   CGCGGATCCGGGGGGGGGGGGCAGAACCACTCCGCCGT
***SEQ ID NO: 42 - oligonucleotide primer***
   CCCAAGCTTCTGTTTGCCGGCGATGCC
***SEQ ID NO: 43 - B_{M1239}C_{MC58}*** 
QNHSAVVALQIEKINNPDKTDSLINQRSFLVSGLGGEHTAFNQLPGGKAEYHGKAFSSDDPNGKLTYTIDFAAKQGNGKIEHLKSPELNVDLAAAD
IKPDGKRHAVISGSVLYN
***SEQ ID NO: 44 - BC₂₉₉₆BC_{M1239}*** 
QDHSAVVALQIEKINNPDKIDSLINQRSFLVSGLGGEHTAFNQLPDGKAEYHGKAFSSDDAGGKLTYTIDFAAKQGHGKIEHLKTPEQNVELAAAE
LKADEKSHAVILGDTRYGSEEKGTYHLALFGDRAQEIAGSATVKIGEKVHEIGIAGKQGSGGGGQNHSAVVALQIEKINNPDKTDSLINQRSFLVS
GLGGEHTAFNQLPGGKAEYHGKAFSSDDPNGRLHYSIDFTKKQGYGRIEHLKTLEQNVELAAAELKADEKSHAVILGDTRYGSEEKGTYHLALFGD
RAQEIAGSATVKIGEKVHEIGIAGKQ
***SEQ ID NO: 45***
   GKGGGG
***SEQ ID NO: 46 - Primer***
   CGCGGATCCCATATGCAAAGCCATTCCGCCTTAA
***SEQ ID NO: 47 - Primer***
   CGCGGATCCTTGCTTGGCGGCAAGGC
***SEQ ID NO: 48 - Primer***
   CGCGGATCCGGGGGGGGGGGGCAGAACCACTCCGCCGT
***SEQ ID NO: 49 - Primer***
   CCCAAGCTTCTGTTTGCCGGCGATGCC
***SEQ ID NO: 50 - Primer***
   CGCGGATCCGGGGGGGGGGGGCAGGACCACTCCGCCG
***SEQ ID NO: 51 - Primer***
   CCCGCTCGAGCTGTTTGCCGGCGATGCC
***SEQ ID NO: 52*** 
QSHSALTAFQTEQIQDSEHSGKMVAKRQFRIGDIAGEHTSFDKLPEGGRATYRGTAFGSDDAGGKLTYTIDFAAKQGNGKIEHLKSPELNVDLAAA
DIKPDGKRHAVISGSVLYNQAEKGSYSLGIFGGKAQEVAGSAEVKTVNGIRHIGLAAKQGSGGGGQNHSAVVALQIEKINNPDKTDSLINQRSFLV
SGLGGEHTAFNQLPGGKAEYHGKAFSSDDPNGRLHYSIDFTKKQGYGRIEHLKTLEQNVELAAAELKADEKSHAVILGDTRYGSEEKGTYHLALFG
DRAQEIAGSATVKIGEKVHEIGIAGKQGKGGGGQDHSAVVALQIEKINNPDKIDSLINQRSFLVSGLGGEHTAFNQLPDGKAEYHGKAFSSDDAGG
KLTYTIDFAAKQGHGKIEHLKTPEQNVELAAAELKADEKSHAVILGDTRYGSEEKGTYHLALFGDRAQEIAGSATVKIGEKVHEIGIAGKQ
***SEQ ID NO: 53*** 
VAADIGAGLADALTAPLDHKDKGLQSLTLDQSVRKNEKLKLAAQGAEKTYGNGDSLNTGKLKNDKVSRFDFIRQIEVDGQLITLESGEFQVYKQSH
SALTAFQTEQIQDSEHSGKMVAKRQFRIGDIAGEHTSFDKLPEGGRATYRGTAFGSDDAGGKLTYTIDFAAKQGNGKIEHLKSPELNVDLAAADIK
PDGKRHAVISGSVLYNQAEKGSYSLGIFGGKAQEVAGSAEVKTVNGIRHIGLAAKQGSGPDSDRLQQRRVAADIGTGLADALTAPLDHKDKGLKSL
TLEDSIPQNGTLTLSAQGAEKTFKAGDKDNSLNTGKLKNDKISRFDFVQKIEVDGQTITLASGEFQIYKQNHSAVVALQIEKINNPDKTDSLINQR
SFLVSGLGGEHTAFNQLPGGKAEYHGKAFSSDDPNGRLHYSIDFTKKQGYGRIEHLKTLEQNVELAAAELKADEKSHAVILGDTRYGSEEKGTYHL
ALFGDRAQEIAGSATVKIGEKVHEIGIAGKQGKGPDSDRLQQRRVAADIGAGLADALTAPLDHKDKSLQSLTLDQSVRKNEKLKLAAQGAEKTYGN
GDSLNTGKLKNDKVSRFDFIRQIEVDGQLITLESGEFQIYKQDHSAVVALQIEKINNPDKIDSLINQRSFLVSGLGGEHTAFNQLPDGKAEYHGKA
FSSDDAGGKLTYTIDFAAKQGHGKIEHLKTPEQNVELAAAELKADEKSHAVILGDTRYGSEEKGTYHLALFGDRAQEIAGSATVKIGEKVHEIGIA
GKQ
***SEQ ID NO: 54 - Sequence for expression***
   GKGPDSDRLQQRR
***SEQ ID NO: 55 - Oligonucleotide primer***
   CGCGGATCCCATATGGTCGCCGCCGACATCG
***SEQ ID NO: 56 - Oligonucleotide primer***
   CGCGGATCCTTGCTTGGCGGCAAGGC
***SEQ ID NO:* 57 - *Oligonucleotide primer***
   CGCGGATCCGGCCCTGATTCTGACCG
***SEQ ID NO: 58 - Oligonucleotide primer***
   CCCAAGCTTCTGTTTGCCGGCGATGCC
***SEQ ID NO: 59 - Oligonucleotide primer***
   CGCGGATCCGGCCCTGATTCTGACCG
***SEQ ID NO: 60 - Oligonucleotide primer***
   CCCGCTCGAGCTGTTTGCCGGCGATGCC
***SEQ ID NO: 61 - Surface loop substitution*** 
MNRTAFCCLSLTTALILTACSSGGGGVAADIGAGLADALTAPLDHKDKGLQSLTLDQSVRKNEKLKLAAQGAEKTYGNGDSLNTGKLKNDKVSRFD
FIRQIEVDGQLITLESGEFQVYKQSHSALTAFQTEQINNPDKIDSMVAKRQFRIGDIAGEHTSFDQLPDGKATYRGTAFGSDDPNGKLTYTIDFAA
KQGHGKIEHLKSPELNVDLAAADIKADEKSHAVISGSVLYGSEEKGSYSLGIFGGKAQEVAGSAEVKIGEKVHEIGLAAKQ
***SEQ ID NO: 62 - strain 4243*** 
CSSGGGGVAADIGAGLADALTAPLDHKDKGLQSLMLDQSVRKNEKLKLAAQGAEKTYGNGDSLNTGKLKNDKVSRFDFIRQIEVDGQLITLESGEF
QVYKQSHSALTALQTEQVQDSEDSGKMVAKRQFRIGDIAGEHTSFDKLPKDVMATYRGTAFGSDDAGGKLTYTIDFAAKQGHGKIEHLKSPELNVD
LAAAYIKPDEKHHAVISGSVLYNQAEKGSYSLGIFGGKAQEVAGSAEVKTVNGIRHIGLAAKQ
***SEQ ID NO: 63 - strain gb320*** 
CSSGSGSGGGGVAADIGTGLADALTAPLDHKDKGLKSLTLEDSISQNGTLTLSAQGAEKTFKVGDKDNSLNTGKLKNDKISRFDFVQKIEVDGQTI
TLASGEFQIYKQDHSAVVALQIEKINNPDKIDSLINQRSFLVSGLGGEHTAFNQLPSGKAEYHGKAFSSDDAGGKLTYTIDFAAKQGHGKIEHLKT
PEQNVELASAELKADEKSHAVILGDTRYGSEEKGTYHLALFGDRAQEIAGSATVKIREKVHEIGIAGKQ
***SEQ ID NO: 64 - strain S10026*** 
CSSGGGGSGGGGVAADIGVGLADALTTPLDHKDKGLQSLTLDQSVRKNEKLKLAAQGAEKTYGNGDSLNTGKLKNDKVSRFDFIRQIEVDGQTITL
ASGEFQIYKQNHSAVVALQIEKINNPDKIDSLINQRSFLVSGLGGEHTAFNQLPDGKAEYHGKAFSSDDPNGRLHYSIDFTKKQGYGRIEHLKTPE
QNVELASAELKADEKSHAVILGDTRYGGEEKGTYHLALFGDRAQEIAGSATVKIREKVHEIGIAGKQ
***SEQ ID NO: 65 - strain m3813*** 
CSSGGGGSGGIAADIGTGLADALTAPLDHKDKGLKSLTLEDSIPQNGTLTLSAQGAEKTFKAGDKDNSLNTGKLKNDKISRFDFVQKIEVDGQTIT
LASGEFQIYKQDHSAVVALQIEKINNPDKIDSLINQRSFLVSGLGGEHTAFNQLPGGKAEYHGKAFSSDDAGGKLTYTIDFAAKQGHGKIEHLKTP
EQNVELAAAELKADEKSHAVILGDTRYGSEEKGTYHLALFGDRAQEIAGSATVKIGEKVHEIGIAGKQ
***SEQ ID NO: 66 - SDM primer***
   GATTTCGCCGCCAAGCAGGGACACGGCAAAATCGAA
***SEQ ID NO:* 67 - *SDM primer***
   TCCCTGCTTGGCGGCGAAATCTATGGTGTAGGT
***SEQ ID NO: 68 - SDMprimer***
   GCCGCCGATATCAAGCCGGATAAAAAACGCCATGCC
***SEQ ID NO: 69 - SDM primer***
   ATCCGGCTTGATATCGGCGGCGGCCAGGTCGAC
***SEQ ID NO: 70 - SDM primer***
   GATATCAAGCCGGATGGAAAACACCATGCCGTCATCAGC
***SEQ ID NO: 71 - SDM primer***
   TTTTCCATCCGGCTTGATATCGGCGGCGGCCAGGTC
***SEQ ID NO: 72 - SDMprimer***
   GCCTTTCAGACCGAGCAAATAAACAACCCGGACAAAATCGACAGCATGGTTGCGAAACGC
***SEQ ID NO: 73 - SDMprimer***
   TATTTGCTCGGTCTGAAAGGCGGTTAAGGCGGA
***SEQ ID NO: 74 - SDM primer***
   GGCGAACATACATCTTTTGACCAGCTTCCCGACGGCAAAAGGGCGACATATCGC
***SEQ ID NO: 75 - SDM primer***
   GTCAAAAGATGTATGTTCGCCCGCTATGTCGCC
***SEQ ID NO: 76 - SDM primer***
   ACGGCGTTCGGTTCAGACGATCCGAACGGAAAACTGACCTAC
***SEQ ID NO: 77- SDM primer***
   ATCGTCTGAACCGAACGCCGTCCCGCGATATGTCGC
***SEQ ID NO: 78 - SDM primer***
   GATTTCGCCGCCAAGCAGGGACACGGCAAAATCGAA
***SEQ ID NO: 79 - SDM primer***
   TCCCTGCTTGGCGGCGAAATCTATGGTGTAGGT
***SEQ ID NO: 80 - SDM primer***
   CTGGCCGCCGCCGATATCAAGGCCGATGAAAAAAGCCATGCCGTCATC
***SEQ ID NO: 81 - SDM primer***
   CTTGATATCGGCGGCGGCCAGGTCGACATTGAG
***SEQ ID NO: 82 - SDM primer***
   ATCAGCGGTTCCGTCCTTTACGGCAGCGAAGAGAAAGGCAGT

## Claims

1. A process for producing a chimeric NMB1870 amino acid sequence, comprising the steps of: (a) aligning a first NMB1870 amino acid sequence with a second NMB1870 amino acid sequence, wherein the first and second sequences are different and are from different NMB 1870 families, to give a pair of aligned sequences; (b) selecting a portion of the first amino acid sequence, starting at amino acid a₁ of said first amino acid sequence and ending at amino acid b₁ of said first amino acid sequence, wherein the portion is a surface loop sequence; (c) selecting a portion of the second amino acid sequence, starting at amino acid a₂ of said second amino acid sequence and ending at amino acid b₂ of said second amino acid sequence, wherein the portion is a surface loop sequence, and wherein residues a₁ & a₂ and b₁ & b₂ are aligned in the pair of aligned sequences; and (d) replacing said portion of the first amino acid sequence with said portion of the second amino acid sequence, thereby providing the chimeric NMB1870 amino acid sequence, wherein the amino acid sequences comprise a backbone sequence , in eight parts, and seven surface loops, one between each part of backbone sequence.

2. The process of claim 1, wherein the first sequence is a family 1 NMB 1870 sequence.

3. The process of claim 2, wherein the first sequence is SEQ ID NO: 1.

4. The process of claim 3, wherein the surface loops in SEQ ID NO: 1 are: (1) amino acids 134-141; (2) amino acids 162-168; (3) amino acids 181-182; (4) amino acid 197; (5) amino acids 219-223; (6) amino acids 234-236; (7) amino acids 261-267:

5. The process of any preceding claim, wherein the second sequence is SEQ ID NO: 2 or SEQ ID NO: 3.

6. The process of any preceding claim, wherein the selected portions are at least 3 amino acids long.

7. The process of any of claims 1-6, wherein the portion comprises surface loop (1).

8. The process of any of claims 1-6, wherein the portion comprises surface loop (2).

9. The process of any of claims 1-5, wherein the portion comprises surface loop (3).

10. The process of any of claims 1-5, wherein the portion comprises surface loop (4).

11. The process of any of claims 1-6, wherein the portion comprises surface loop (5).

12. The process of any of claims 1-6, wherein the portion comprises surface loop (6).

13. The process of any of claims 1-6, wherein the portion comprises surface loop (7).

14. A chimeric polypeptide comprising a chimeric NMB 1870 amino acid sequence obtainable by the process of any one of claims 1 to 5.

15. A polypeptide comprising an amino acid sequence:
-B₁-L₁-B₂-L₂-B₃-L₃-B₄-L₄-B₅-L₅-B₆-L₆-B₇-L₇-B₈-
wherein:
I (a) each of said B₁, B₂, B₃, B₄, B₅, B₆, B₇ and B₈ is: (i) a fragment of SEQ ID NO: 1; and is (ii) an amino acid sequence having at least 95% sequence identity to said fragment of (i) and/or comprising a fragment of at least 6 contiguous amino acids from said fragment of (i);
(b) each of said L₁, L₂, L₃, L₄, L₅, L₆ and L₇ is: (iii) a fragment of SEQ ID NO: 1, SEQ ID NO: 2 and/or of SEQ ID NO: 3, provided that at least one of said L₁, L₂, L₃, L₄, L₅, L₆ and L₇ is not a fragment of SEQ ID NO: 1,
and wherein B₁ to B₈ are defined as follows:
| **Amino acid co-ordinates within SEQ ID NO: 1** | | | | | | | |
|---|---|---|---|---|---|---|---|
| B₁ | B₂ | B₃ | B₄ | B₅ | B₆ | B₇ | B₈ |
| 1-133 | 142-161 | 169-180 | 183-196 | 198-218 | 224-233 | 237-260 | 268-274 |
and wherein L₁ to L₇ are defined as follows:
| | **Amino acid co-ordinates within SEQ ID NO: 1, 2 or 3** | | | | | | |
|---|---|---|---|---|---|---|---|
| ***SEQ*** | L₁ | L₂ | L₃ | L₄ | L₅ | L₆ | L₇ |
| **1** | 134-141 | 162-168 | 181-182 | 197 | 219-223 | 234-236 | 261-267 |
| **2** | 134-141 | 162-167 | 180-181 | 196 | 218-222 | 233-235 | 260-266 |
| **3** | 142-149 | 170-175 | 188-189 | 204 | 226-230 | 241-243 | 268-274 |

16. A polypeptide comprising an amino acid sequence that has an overall sequence identity to SEQ ID NO: 1 of at least 80%, wherein: the sequence identity of said amino acid sequence to SEQ ID NO: 1 is more than 80% at the backbone regions of SEQ ID NO: 1; and the sequence identity of said amino acid sequence to SEQ ID NO: 1 is less than 80% at the loop regions of SEQ ID NO: 1, wherein the amino acid sequence comprises a backbone, in eight backbone regions, and seven loop regions, one between each part of backbone sequence.

17. A polypeptide comprising an amino acid sequence that has an overall sequence identity to SEQ ID NO: 2 of at least 80%, wherein: the sequence identity of said amino acid sequence to SEQ ID NO: 2 is more than 80% at the backbone regions of SEQ ID NO: 2; and the sequence identity of said amino acid sequence to SEQ ID NO: 2 is less than 80% at the loop regions of SEQ ID NO: 2, wherein the amino acid sequence comprises a backbone, in eight backbone regions, and seven loop regions, one between each part of backbone sequence.

18. A polypeptide comprising an amino acid sequence that has an overall sequence identity to SEQ ID NO: 3 of at least 80%, wherein: the sequence identity of said amino acid sequence to SEQ ID NO: 3 is more than 80% at the backbone regions of SEQ ID NO: 3; and the sequence identity of said amino acid sequence to SEQ ID NO: 3 is less than 80% at the loop regions of SEQ ID NO: 3, wherein the amino acid sequence comprises a backbone, in eight backbone regions, and seven loop regions, one between each part of backbone sequence.

19. Nucleic acid encoding a polypeptide of any one of claims 14 to 18.

20. An immunogenic composition, comprising the polypeptide of any one of claims 14 to 18.

21. The composition of claim 20, further comprising an aluminium salt adjuvant.

22. The composition of claim 20 or claim 21, further comprising a meningococcal PorA protein.

23. The composition of claim 20 or claim 21, further comprising an outer membrane vesicle preparation from *N.meningitidis.*

24. The polypeptide of any one of claims 14 to 18, for use as a medicament.

## Patentansprüche

1. Verfahren zur Herstellung einer chimären NMB1870-Aminosäuresequenz, mit den folgenden Schritten:
(a) Ausrichten einer ersten NMB1870-Aminosäuresequenz an einer zweiten NMB1870-Aminosäuresequenz, wobei die erste und die zweite Sequenz unterschiedlich sind und aus unterschiedlichen NMB1870-Familien stammen, so dass man ein Paar aneinander ausgerichteter Sequenzen erhält; (b) Auswählen eines Anteils der ersten Aminosäuresequenz, beginnend mit Aminosäure a₁ der ersten Aminosäuresequenz und endend mit Aminosäure b₁ der ersten Aminosäuresequenz, wobei es sich bei dem Anteil um eine Oberflächen-Loop-Sequenz handelt;
(c) Auswählen eines Anteils der zweiten Aminosäuresequenz, beginnend mit Aminosäure a₂ der zweiten Aminosäuresequenz und endend mit Aminosäure b₂ der zweiten Aminosäuresequenz, wobei es sich bei dem Anteil um eine Oberflächen-Loop-Sequenz handelt und wobei die Reste a₁ & a₂ sowie b₁ & b₂ in dem Paar aneinander ausgerichteter Sequenzen ausgerichtet sind; und (d) Ersetzen des Anteils der ersten Aminosäuresequenz durch den Anteil der zweiten Aminosäuresequenz, wodurch die chimäre NMB1870-Aminosäuresequenz bereitgestellt wird, wobei die Aminosäuresequenzen eine Grundgerüstsequenz in acht Teilen sowie sieben Oberflächen-Loops, von denen jeweils eine zwischen den einzelnen Teilen der Grundgerüstsequenz liegt, umfassen.

2. Verfahren nach Anspruch 1, wobei es sich bei der ersten Sequenz um eine NMB1870-Sequenz der Familie I handelt.

3. Verfahren nach Anspruch 2, wobei es sich bei der ersten Sequenz um SEQ ID NO: 1 handelt.

4. Verfahren nach Anspruch 3, wobei es sich bei den Oberflächen-Loops in SEQ ID NO: 1 um (1) Aminosäuren 134-141; (2) Aminosäuren 162-168; (3) Aminosäuren 181-182; (4) Aminosäure 197; (5) Aminosäuren 219-223; (6) Aminosäuren 234-236; (7) Aminosäuren 261-267 handelt.

5. Verfahren nach einem vorhergehenden Anspruch, wobei es sich bei der zweiten Sequenz um SEQ ID NO: 2 oder SEQ ID NO: 3 handelt.

6. Verfahren nach einem vorhergehenden Anspruch, wobei die ausgewählten Anteile eine Länge von wenigstens 3 Aminosäuren aufweisen.

7. Verfahren nach einem der Ansprüche 1-6, wobei der Anteil Oberflächen-Loop (1) umfasst.

8. Verfahren nach einem der Ansprüche 1-6, wobei der Anteil Oberflächen-Loop (2) umfasst.

9. Verfahren nach einem der Ansprüche 1-5, wobei der Anteil Oberflächen-Loop (3) umfasst.

10. Verfahren nach einem der Ansprüche 1-5, wobei der Anteil Oberflächen-Loop (4) umfasst.

11. Verfahren nach einem der Ansprüche 1-6, wobei der Anteil Oberflächen-Loop (5) umfasst.

12. Verfahren nach einem der Ansprüche 1-6, wobei der Anteil Oberflächen-Loop (6) umfasst.

13. Verfahren nach einem der Ansprüche 1-6, wobei der Anteil Oberflächen-Loop (7) umfasst.

14. Chimäres Polypeptid, umfassend eine mit dem Verfahren nach einem der Ansprüche 1 bis 5 erhältliche chimäre NMB1870-Aminosäuresequenz.

15. Polypeptid, umfassend eine Aminosäuresequenz:
-B₁-L₁-B₂-L₂-B₃-L₃-B₄-L₄-B₅-L₅-B₆-L₆-B₇-L₇-B₈-
wobei:
(a) B₁, B₂, B₃, B₄, B₅, B₆, B₇ und B₈ jeweils für (i) ein Fragment von SEQ ID NO: 1 und/oder (ii) eine Aminosäuresequenz, die eine Sequenzidentität von wenigstens 95% mit dem Fragment unter (i) aufweist und/oder ein Fragment von wenigstens 6 aufeinander folgenden Aminosäuren aus dem Fragment unter (i) umfasst, stehen;
(b) L₁, L₂, L₃, L₄, L₅, L₆ und L₇ jeweils für (iii) ein Fragment von SEQ ID NO: 1, SEQ ID NO: 2 und/oder von SEQ ID NO: 3 stehen, vorausgesetzt, dass wenigstens eine der Sequenzen L₁, L₂, L₃, L₄, L₅, L₆ und L₇ kein Fragment von SEQ ID NO: 1 ist, und wobei B₁ bis B₈ die folgende Bedeutung haben:
| **Aminosäurekoordinaten in SEQ ID NO: 1** | | | | | | | |
|---|---|---|---|---|---|---|---|
| B₁ | B₂ | B₃ | B₄ | B₅ | B₆ | B₇ | B₈ |
| 1-133 | 142-161 | 169-180 | 183-196 | 198-218 | 224-233 | 237-260 | 268-274 |
und wobei L₁ bis L₇ die folgende Bedeutung haben:
| **Aminosäurekoordinaten in SEQ ID NO: 1, 2 oder 3** | | | | | | | |
|---|---|---|---|---|---|---|---|
| ***SEQ*** | L₁ | L₂ | L₃ | L₄ | L₅ | L₆ | L₇ |
| **1** | 134-141 | 162-168 | 181-182 | 197 | 219-223 | 234-236 | 261-267 |
| **2** | 134-141 | 162-167 | 180-181 | 196 | 218-222 | 233-235 | 260-266 |
| **3** | 142-149 | 170-175 | 188-189 | 204 | 226-230 | 241-243 | 268-274 |

16. Polypeptid, umfassend eine Aminosäuresequenz, die eine Gesamtsequenzidentität von wenigstens 80% mit SEQ ID NO: 1 aufweist, wobei: die Sequenzidentität der Aminosäuresequenz mit SEQ ID NO: 1 bei den Grundgerüstbereichen von SEQ ID NO: 1 mehr als 80% beträgt; und die Sequenzidentität der Aminosäuresequenz mit SEQ ID NO: 1 bei den Loop-Bereichen von SEQ ID NO: 1 weniger als 80% beträgt, wobei die Aminosäuresequenz ein Grundgerüst, in acht Grundgerüstbereichen, sowie sieben Loop-Bereiche, von denen jeweils einer zwischen den einzelnen Teilen der Grundgerüstsequenz liegt, umfasst.

17. Polypeptid, umfassend eine Aminosäuresequenz, die eine Gesamtsequenzidentität von wenigstens 80% mit SEQ ID NO: 2 aufweist, wobei: die Sequenzidentität der Aminosäuresequenz mit SEQ ID NO: 2 bei den Grundgerüstbereichen von SEQ ID NO: 2 mehr als 80% beträgt; und die Sequenzidentität der Aminosäuresequenz mit SEQ ID NO: 2 bei den Loop-Bereichen von SEQ ID NO: 2 weniger als 80% beträgt, wobei die Aminosäuresequenz ein Grundgerüst, in acht Grundgerüstbereichen, sowie sieben Loop-Bereiche, von denen jeweils einer zwischen den einzelnen Teilen der Grundgerüstsequenz liegt, umfasst.

18. Polypeptid, umfassend eine Aminosäuresequenz, die eine Gesamtsequenzidentität von wenigstens 80% mit SEQ ID NO: 3 aufweist, wobei: die Sequenzidentität der Aminosäuresequenz mit SEQ ID NO: 3 bei den Grundgerüstbereichen von SEQ ID NO: 3 mehr als 80% beträgt; und die Sequenzidentität der Aminosäuresequenz mit SEQ ID NO: 3 bei den Loop-Bereichen von SEQ ID NO: 3 weniger als 80% beträgt, wobei die Aminosäuresequenz ein Grundgerüst, in acht Grundgerüstbereichen, sowie sieben Loop-Bereiche, von denen jeweils einer zwischen den einzelnen Teilen der Grundgerüstsequenz liegt, umfasst.

19. Nukleinsäure, codierend für ein Polypeptid nach einem der Ansprüche 14 bis 18.

20. Immunogene Zusammensetzung, umfassend das Polypeptid nach einem der Ansprüche 14 bis 18.

21. Zusammensetzung nach Anspruch 20, ferner umfassend ein Aluminiumsalz-Adjuvans.

22. Zusammensetzung nach Anspruch 20 oder Anspruch 21, ferner umfassend ein Meningokokken-PorA-Protein.

23. Zusammensetzung nach Anspruch 20 oder Anspruch 21, ferner umfassend eine Präparation von Vesikeln der äußeren Membran aus *N*. *meningitidis.*

24. Polypeptid nach einem der Ansprüche 14 bis 18, zur Verwendung als Arzneimittel.

## Revendications

1. Processus pour produire une séquence d'acides aminés NMB1870 chimère, comprenant les étapes consistant à : (a) aligner une première séquence d'acides aminés NMB1870 avec une deuxième séquence d'acides aminés NMB1870, dans laquelle les première et deuxième séquences sont différentes et proviennent de familles différentes de NMB1870, pour donner une paire de séquences alignées ; (b) choisir une partie de la première séquence d'acides aminés, en commençant au niveau de l'acide aminé a₁ de ladite première séquence d'acides aminés et en finissant au niveau de l'acide aminé b₁ de ladite première séquence d'acides aminés, dans laquelle la partie est une séquence de surface en boucle ; (c) choisir une partie de la deuxième séquence d'acides aminés, en commençant au niveau de l'acide aminé a₂ de ladite deuxième séquence d'acides aminés et en finissant au niveau de l'acide aminé b₂ de ladite deuxième séquence d'acides aminés, dans laquelle la partie est une séquence de surface en boucle et dans laquelle les résidus a₁ & a₂ ainsi que b₁ & b₂ sont alignés dans la paire de séquences alignées ; et (d) remplacer ladite partie de la première séquence d'acides aminés par ladite partie de la deuxième séquence d'acides aminés, moyennant quoi cela fournit la séquence d'acides aminés NMB1870 chimère, dans laquelle les séquences d'acides aminés comprennent une séquence squelette, en huit parties et sept boucles de surface, une entre chaque partie de la séquence squelette.

2. Processus selon la revendication 1, dans lequel la première séquence est une séquence NMB1870 de la famille I.

3. Processus selon la revendication 2, dans lequel la première séquence est la SEQ ID n° : 1.

4. Processus selon la revendication 3, dans lequel les boucles de surface dans la SEQ ID n° : 1 sont : (1) les acides aminés 134 à 141 ; (2) les acides aminés 162 à 168 ; (3) les acides aminés 181 à 182 ; (4) l'acide aminé 197 ; (5) les acides aminés 219 à 223 ; (6) les acides aminés 234 à 236 ; (7) les acides aminés 261 à 267.

5. Processus selon l'une quelconque des revendications précédentes, dans lequel la deuxième séquence est la SEQ ID n° : 2 ou la SEQ ID n° : 3.

6. Processus selon l'une quelconque des revendications précédentes, dans lequel les parties choisies ont une longueur d'au moins 3 acides aminés.

7. Processus selon l'une quelconque des revendications 1 à 6, dans lequel la partie comprend la boucle de surface (1).

8. Processus selon l'une quelconque des revendications 1 à 6, dans lequel la partie comprend la boucle de surface (2).

9. Processus selon l'une quelconque des revendications 1 à 5, dans lequel la partie comprend la boucle de surface (3).

10. Processus selon l'une quelconque des revendications 1 à 5, dans lequel la partie comprend la boucle de surface (4).

11. Processus selon l'une quelconque des revendications 1 à 6, dans lequel la partie comprend la boucle de surface (5).

12. Processus selon l'une quelconque des revendications 1 à 6, dans lequel la partie comprend la boucle de surface (6).

13. Processus selon l'une quelconque des revendications 1 à 6, dans lequel la partie comprend la boucle de surface (7).

14. Polypeptide chimère comprenant une séquence d'acides aminés NMB1870 chimère, que l'on peut obtenir par le processus selon l'une quelconque des revendications 1 à 5.

15. Polypeptide comprenant une séquence d'acides aminés :
-B₁-L₁-B₂-L₃-B₃-L₃-B₄-L₄-B₅-L₅-B₆-L₆-B₇-L₇-B₈-
dans laquelle :
(a) chacun desdits B₁, B₂, B₃, B₄, B₅, B₆, B₇ et B₈ est : (i) un fragment de la SEQ ID n° : 1 et/ou (ii) une séquence d'acides aminés ayant une identité de séquence d'au moins 95% avec ledit fragment selon (i) et/ou comprenant un fragment d'au moins 6 acides aminés contigus provenant dudit fragment selon (i) ;
(b) chacun desdits L₁, L₂, L₃, L₄, L₅, L₆ et L₇ est : (iii) un fragment de la SEQ ID n° : 1, SEQ ID n° : 2 et/ou SEQ ID n° : 3, à condition qu'au moins l'un desdits L₁, L₂, L₃, L₄, L₅, L₆ et L₇ ne soit pas un fragment de la SEQ ID n° : 1,
et dans laquelle B₁ à B₈ sont définis ainsi qu'il suit :
| **Coordonnées d'acides aminés au sein de la SEQ ID n° : 1** | | | | | | | |
|---|---|---|---|---|---|---|---|
| B₁ | B₂ | B₃ | B₄ | B₅ | B₆ | B₇ | B₈ |
| 1-133 | 142-161 | 169-180 | 183-196 | 198-218 | 224-233 | 237-260 | 268-274 |
et dans laquelle L₁ à L₇ sont définis ainsi qu'il suit :
| | **Coordonnées d'acides aminés au sein des SEQ ID n° : 1, 2 ou 3** | | | | | | |
|---|---|---|---|---|---|---|---|
| ***SEQ*** | L₁ | L₂ | L₃ | L₄ | L₅ | L₆ | L₇ |
| **1** | 134-141 | 162-168 | 171-182 | 197 | 219-223 | 234-236 | 261-267 |
| **2** | 134-141 | 162-167 | 180-181 | 196 | 218-222 | 233-235 | 260-266 |
| **3** | 142-149 | 170-175 | 188-189 | 204 | 226-230 | 241-243 | 268-274 |

16. Polypeptide comprenant une séquence d'acides aminés qui a une identité de séquence globale avec la SEQ ID n° : 1 d'au moins 80%, dans laquelle : l'identité de séquence de ladite séquence d'acides aminés avec la SEQ ID n° : 1 est supérieure à 80% au niveau des régions squelettes de la SEQ ID n° : 1 et l'identité de séquence de ladite séquence d'acides aminés avec la SEQ ID n° : 1 est inférieure à 80% au niveau des régions en boucle de la SEQ ID n° : 1, dans laquelle la séquence d'acides aminés comprend un squelette, en huit régions squelettes, et sept régions en boucle, une entre chaque partie de la séquence squelette.

17. Polypeptide comprenant une séquence d'acides aminés qui a une identité de séquence globale avec la SEQ ID n° : 2 d'au moins 80%, dans laquelle : l'identité de séquence de ladite séquence d'acides aminés avec la SEQ ID n° : 2 est supérieure à 80% au niveau des régions squelettes de la SEQ ID n° : 2 et l'identité de séquence de ladite séquence d'acides aminés avec la SEQ ID n° : 2 est inférieure à 80% au niveau des régions en boucle de la SEQ ID n° : 2, dans laquelle la séquence d'acides aminés comprend un squelette, en huit régions squelettes, et sept régions en boucle, une entre chaque partie de la séquence squelette.

18. Polypeptide comprenant une séquence d'acides aminés qui a une identité de séquence globale avec la SEQ ID n° : 3 d'au moins 80%, dans laquelle : l'identité de séquence de ladite séquence d'acides aminés avec la SEQ ID n° : 3 est supérieure à 80% au niveau des régions squelettes de la SEQ ID n° : 3 et l'identité de séquence de ladite séquence d'acides aminés avec la SEQ ID n° : 3 est inférieure à 80% au niveau des régions en boucle de la SEQ ID n° : 3, dans laquelle la séquence d'acides aminés comprend un squelette, en huit régions squelettes, et sept régions en boucle, une entre chaque partie de la séquence squelette.

19. Acide nucléique codant pour un polypeptide selon l'une quelconque des revendications 14 à 18.

20. Composition immunogène comprenant le polypeptide selon l'une quelconque des revendications 14 à 18.

21. Composition, selon la revendication 20, comprenant en outre un adjuvant à sel d'aluminium.

22. Composition, selon la revendication 20 ou la revendication 21, comprenant en outre une protéine PorA méningococcique.

23. Composition, selon la revendication 20 ou la revendication 21, comprenant en outre une préparation de vésicule de la membrane externe provenant de N. *meningitidis.*

24. Polypeptide, selon l'une quelconque des revendications 14 à 18, destiné à être utilisé comme médicament.
